# DEMANDE DE BREVET EUROPEEN

(11) **EP 1 346 992 A1**
(43) Date de publication de la demande: **24.09.2003**
(21) Numéro de dépôt: 03290663.8
(22) Date de dépôt: 17.03.2003
(51) Int. Cl.: C07D 403/06, C07D 401/14, C07D 405/14, C07D 409/14, A61K 31/517, A61P 35/00

(54) **Nouveaux composés dérivés de la quinazoline, leur procédé de préparation et les compositions pharmaceutiques qui les contiennent**

(30) Priorité: 18.03.2002 FR 0203299
(71) Demandeur: Les Laboratoires Servier, 92415 Courbevoie Cedex (FR)
(72) Inventeur: Goldstein, Solo, 92150 Suresnes (FR); Dhainaut, Alain, 78400 Chatou (FR); Tizot, André, 91370 Verrieres-le-Buison (FR); Fauchere, Jean-Luc, 92210 Saint-Cloud (FR); Kucharczyk, Nathalie, 78112 Fourqueux (FR); Hickman, John, 75017 Paris (FR); Pierre, Alain, 78580 Les Alluets le Roi (FR); Tucker, Gordon, 75017 Paris (FR); Kraus-Berthier, Laurence, 92700 Colombes (FR)

(57) **Abrégé**

Composés de formule (I) : 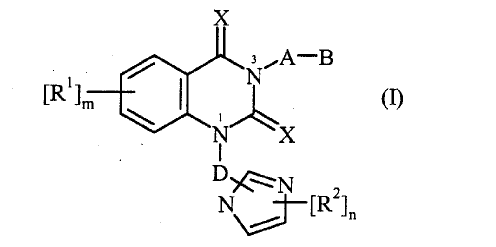 dans laquelle :
A, B, D, X, R¹, R², m et n sont tels que définis dans la description, leurs stéréoisomères ainsi que leurs sels d'addition à un acide ou à une base pharmaceutiquement acceptable.

## Description

La présente invention concerne de nouveaux composés dérivés de la quinazoline, leur procédé de préparation et les compositions pharmaceutiques qui les contiennent.

Les composés de l'invention sont utiles comme inhibiteurs de farnésyltransférase.

De nombreuses protéines subissent des altérations post-traductionnelles qui altèrent leur localisation et leur fonction. En particulier, des modifications de type lipidique permettent l'ancrage dans la membrane plasmique de certaines protéines inactives sous leur forme libre, une étape cruciale pour assurer leur fonction. C'est le cas de la prénylation *(Curr. Opin. Cell. Biol., 4, 1992, 1008-1016*) qui est catalysée par plusieurs enzymes: la farnésyltransférase (FTase) et les deux géranylgéranyltransférases (GGTase-I et GGTase-II) qui accrochent un groupe prényle à 15 (*trans,trans*-farnésyl) ou 20 carbones (all-*trans*géranylgéranyl) sur la partie carboxyterminale des protéines substrats (*J. Biol. Chem., 271, 1996, 5289-5292 ; Curr. Opin. Struct. Biol., 7, 1997, 873-880).* La FTase catalyse ce transfert à partir de farnésyl pyrophosphate pour former un lien thioéther sur la cystéine de la séquence tétrapeptidique terminale consensus CA₁A₂X retrouvée sur les substrats protéines, où C désigne la cystéine, A₁ et A₂ un acide aminé aliphatique et X une sérine, une alanine ou une méthionine. La GGTase-I utilise du géranylgéranyl pyrophosphate comme substrat donneur pour effectuer un transfert similaire, mais cette fois la séquence consensus CAAX se termine par une leucine ou une phénylalanine. Ces deux enzymes hétérodimériques partagent une sous-unité alpha de 48 kDa, et possèdent deux chaînes bêta distinctes, quoique présentant 30% d'homologie de séquence en acides aminés. La GGTase-II agit sur des séquences terminales de types XXCC et XCXC et possède des sous-unités alpha et bêta différentes de celles des enzymes précédentes.

L'intérêt d'inhiber une de ces enzymes, la FTase a pour origine l'implication de l'oncogène prénylé Ras dans la progression tumorale (*Annu. Rev. Biochem., 56, 1987, 779-827*). Les protéines Ras existent sous quatre formes majoritaires, Harvey ou H-Ras, N-Ras et Kirsten ou K-Ras A et B. Ces protéines sont exprimées sous une forme mutée dans au moins un quart des cancers avec une incidence encore plus forte pour certains types histologiques de tumeurs et selon la forme de Ras. Par exemple, des mutations de K-Ras B sont retrouvées dans 80 à 90% des carcinomes pancréatiques et 30 à 60% des cancers du côlon (*Int. J. Oncol., 7, 1995, 413-421*). De nombreuses données précliniques ont démontré le rôle de cet oncogène dans la progression tumorale, plus particulièrement dans les phénomènes de croissance cellulaire. Il s'agit d'un lien essentiel dans la transmission des signaux extracellulaires - comme ceux activés par les facteurs de croissance - vers diverses kinases cytosoliques puis vers le noyau, pour une intégration en terme de prolifération, mort cellulaire et survie cellulaire (*Cancer Met. Rev., 13, 1994, 67-89 ; Curr. Opin. Genetics & Develop., 8, 1998, 49-54 ; J. Biol. Chem. 273, 1998, 19925-19928*), ou de régulation avec l'environnement tumoral - angiogenèse en particulier (*Cancer Res., 55, 1995, 4575-4580).*

La recherche d'inhibiteurs de la FTase présente donc un intérêt majeur en oncologie *(Curr. Opin. Chem. Biol., 2, 1998, 40-48).* Comme 0.5% des protéines animales sont vraisemblablement prénylées et en majorité géranylgéranylées, des inhibiteurs spécifiques de la FTase par rapport aux GGTases, et plus particulièrement la GGTase-I, proche en structure de la FTase, sont d'un intérêt majeur. Les premiers travaux avec de tels inhibiteurs, des analogues peptidomimétiques de la séquence consensus de farnésylation, et les suivants avec des molécules issues de criblage de chimiothèques ont validé cette stratégie antitumorale lors d'expérimentations in vitro et chez l'animal (*Annu. Rev. Pharmacol. Toxicol., 37, 1997, 143-166; Biochim. Biophys. Acta, 1423, 1999, C19-C30; Cancer Res., 58, 1998, 4947-4956*). Des fibroblastes spécialement transfectés avec le gène de la protéine H-Ras mutée et implantés chez l'animal développent une masse tumorale dont la croissance est diminuée en fonction de la dose d'inhibiteur de FTase reçue par l'animal. Pour des animaux transgéniques qui expriment une forme mutée de H-Ras sous le contrôle d'un promoteur approprié assurant l'apparition aléatoire de tumeurs salivaires ou mammaires spontanées, ces mêmes inhibiteurs entraînent la régression des tumeurs établies et bloquent l'apparition de nouvelles pendant la durée du traitement. Enfin, ces produits sont aussi actifs pour diminuer la croissance de xénogreffes humaines chez la souris, avec un effet possible d'augmentation de la survie, selon le modèle. La protéine Ras mutée n'est pas la seule cible indirecte de ces inhibiteurs dans la pathologie tumorale (*The Lancet Oncology, 2*, *2001, 18-26; Cell. Mol. Life Sci., 58, 2001, 1636-1649).* L'étude de multiples modèles tumoraux a permis de constater une inhibition de la croissance tumorale indépendamment de la présence de protéines Ras mutées. Cet effet pourrait être en partie lié à une activité directe antiangiogénique et donc indépendante du profil oncogénique de la tumeur (*Eur. J. Cancer, 35, 1999, 1394-1401*). Cette observation renforce et élargit le potentiel d'utilisation antitumorale de cette classe d'inhibiteurs et l'absence d'effet secondaire rédhibitoire sur les fonctions cellulaires normales est aussi favorable à l'inhibition de la FTase dans toute pathologie associée à des mécanismes altérés ou amplifiés par une ou des protéines farnésylées. C'est notamment le cas, par exemple, en dehors du cancer, de la resténose après angioplastie ou chirurgie vasculaire, et de la neurofibromatose de type I (*Mol. Cell. Biol., 17, 1997, 862-872*).

Les composés de l'invention présentent une structure originale et ont le pouvoir d'inhiber sélectivement la FTase vis à vis des GGTases. Ils seront donc utiles dans le traitement de toutes les pathologies associées à une signalisation intracellulaire passant par les protéines Ras ou d'autres protéines farnésylées, et dans les pathologies associées à une amplification de l'angiogenèse. Ainsi ils seront utiles dans le traitement du cancer, mais aussi de la resténose après angioplastie ou chirurgie vasculaire, et de la neurofibromatose de type I.

La présente invention concerne plus particulièrement les composés de formule (I) : 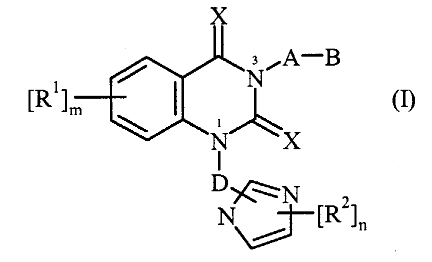 dans laquelle :
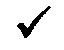 A représente une liaison, un groupement alkylène, alkénylène, alkynylène, T, *-A₁-T-, *-T-A₁-, *-A₁-T-A'₁- ou *-A₁-T-A'₁-T'- (dans lesquels T et T', identiques ou différents, représentent un groupement carbonyle, carbonyloxy, thio, sulfinyle, sulfonyle, oxy, amino, aminoalkyle, aminoaryle, carbonylamino, carbonylaminoalkyle, carbonylaminoaryle, oxycarbonyle, aminocarbonyle, aminoalkylcarbonyle, aminoarylcarbonyle, sulfonylamino, sulfonylaminoalkyle, sulfonylaminoaryle, aminosulfonyle, aminoalkylsulfonyle ou aminoarylsulfonyle ; A₁ et A'₁, identiques ou différents, représentent un groupement alkylène, alkénylène ou alkynylène ; et le symbole "*" représente le point de rattachement à l'atome d'azote N³ du cycle quinazoline),
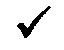 B représente un groupement alkyle éventuellement substitué, aryle éventuellement substitué, hétéroaryle éventuellement substitué, cycloalkyle éventuellement substitué, hétérocycloalkyle éventuellement substitué, arylaminoaryle éventuellement substitué ou arylalkylaryle éventuellement substitué,
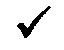 D représente un groupement alkylène dont un atome de carbone de la chaîne hydrocarbonée peut être substitué par un groupement alkyle éventuellement substitué, aryle éventuellement substitué, hétéroaryle éventuellement substitué, cycloalkyle éventuellement substitué, hétérocycloalkyle éventuellement substitué, arylalkyle éventuellement substitué, hétéroarylalkyle éventuellement substitué ou cycloalkylalkyle éventuellement substitué,
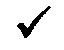 X représente un atome d'oxygène ou de soufre,
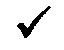 R¹ représente un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
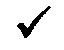 R² représente un atome d'hydrogène, un groupement alkyle, aryle, hétéroaryle, cycloalkyle, hétérocycloalkyle, arylalkyle, hétéroarylalkyle, cycloalkylalkyle ou hétérocycloalkylakyle, tous ces groupements pouvant éventuellement être substitués par un substituant choisi parmi un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle, alkoxycarbonylamino, arylamino éventuellement substitué ou arylalkyle éventuellement substitué,
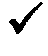 m représente un entier compris inclusivement entre 0 et 4,
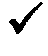 n représente un entier compris inclusivement entre 0 et 3,
leurs énantiomères, diastéréoisomères, ainsi que leurs sels d'addition à un acide ou à une base pharmaceutiquement acceptable,
étant entendu que :
- pour m supérieur à 1, les groupements R¹ peuvent être identiques ou différents les uns des autres,
- pour n supérieur à 1, les groupements R² peuvent être identiques ou différents les uns des autres,
- lorsque les deux atomes d'azote du groupement imidazolyle sont substitués, le groupement imidazolyle devient un groupement cationique imidazolinium,
- le terme alkyle désigne une chaîne hydrocarbonée, linéaire ou ramifiée, contenant de 1 à 6 atomes de carbone,
- le terme alkoxy désigne un groupement alkyle-oxy, linéaire ou ramifié, contenant de 1 à 6 atomes de carbone,
- le terme alkylène désigne une chaîne hydrocarbonée bivalente, linéaire ou ramifiée, contenant de 1 à 6 atomes de carbone,
- le terme alkénylène désigne une chaîne hydrocarbonée bivalente linéaire ou ramifiée, contenant de 1 à 3 doubles liaisons et de 2 à 6 atomes de carbone,
- le terme alkynylène désigne une chaîne hydrocarbonée bivalente linéaire ou ramifiée, contenant de 1 à 3 triples liaisons et de 2 à 6 atomes de carbone,
- le terme cycloalkyle désigne un groupement mono ou polycyclique, saturé ou partiellement saturé, contenant de 3 à 10 atomes de carbone,
- le terme hétérocycloalkyle désigne un groupement mono ou polycyclique saturé ou partiellement insaturé de 5 à 7 chaînons contenant de 1 à 3 hétéroatomes choisis parmi l'azote, l'oxygène et le soufre,
- le terme aryle désigne un groupement phényle, naphtyle ou biphényle,
- le terme hétéroaryle désigne un groupement mono ou bicyclique, aromatique ou contenant au moins un cycle aromatique, de 5 à 11 chaînons, contenant de 1 à 3 hétéroatomes choisis parmi l'azote, l'oxygène ou le soufre,
- le terme éventuellement substitué, affecté à l'expression alkyle, signifie qu'un à trois atomes de carbone de la chaîne hydrocarbonée peuvent être substitués par un à trois substituants, identiques ou différents, choisis parmi atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
- le terme éventuellement substitué, sauf indication contraire, affecté aux expressions aryle, hétéroaryle, cycloalkyle, hétérocycloalkyle, arylalkylaryle et arylaminoaryle, signifie que la ou les parties cycliques de ces groupements peuvent être substituées par un à trois substituants, identiques ou différents, choisi parmi un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
- le terme perhalogénoalkyle désigne un groupement méthyle, éthyle, propyle ou butyle substitué par de 1 à 9 atomes d'halogènes.

Parmi les groupements cycloalkyle, on peut citer les groupements cyclopropyle, cyclobutyle, cyclopentyle, cyclohexyle, bicyclo[2,2,1]heptyle, adamantyle, ...

Parmi les groupements hétéroaryle, on peut citer les groupements pyridyle, furyle, thiényle, indolyle, ...

Parmi les groupements hétérocycloalkyle, on peut citer les groupements pipéridinyle, pipérazinyle, morpholino, ...

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer l'hydroxyde de sodium, l'hydroxyde de potassium, la triéthylamine, la tertbutylamine, etc...

Plus particulièrement, l'invention concerne les composés de formule (I) pour lesquels A représente une liaison, un groupement sulfonyle ou alkylène.

De façon avantageuse, l'invention concerne les composés de formule (I) pour lesquels B représente un groupement aryle éventuellement substitué ou hétéroaryle éventuellement substitué.

De façon préférentielle, dans les composés de formule (I), m vaut 0.

Des composés préférés de l'invention sont ceux pour lesquels X représente un atome d'oxygène.

Des composés préférés de l'invention sont ceux pour lesquels D représente un groupement alkylène substitué par un groupement aryle éventuellement substitué ou arylalkyle éventuellement substitué.

D'autres composés préférés de formule (I) sont ceux pour lesquels R² représente un atome d'hydrogène, un groupement alkyle, ou arylakyle éventuellement substitué.

Dans les composés préférés de formule (I), n vaut 0, 1 ou 2.

La présente invention concerne plus particulièrement les composés de formule (I) pour lesquels m vaut 0, X représente un atome d'oxygène, A représente une liaison, un groupement sulfonyle ou alkylène, B représente un groupement phényle éventuellement substitué, benzylphényle, pyridyle, anilinophényle ou tiényle, D représente un groupement alkylène substitué ou non par un groupement phényle éventuellement substitué ou phénylméthyle éventuellement substitué, n vaut 0, 1 ou 2 et R² représente un groupement alkyle ou arylalkyle éventuellement substitué.

La présente invention concerne plus particulièrement les composés de formule (I) pour lesquels m vaut 0, X représente un atome d'oxygène, A représente une liaison, un groupement sulfonyle ou alkylène, B représente un groupement phényle éventuellement substitué, benzylphényle, pyridyle, anilinophényle ou tiényle, D représente un groupement alkylène substitué ou non par un groupement phényle éventuellement substitué ou phénylméthyle éventuellement substitué, relié à un des atomes d'azote de l'imidazolyle, n vaut 0, 1 ou 2 et R² représente un groupement alkyle ou arylalkyle éventuellement substitué.

L'invention concerne tout particulièrement les composé suivants : 4-(1-[2-benzyl-3-(1*H*-imidazol-1-yl)propyl]-2,4-dioxo-1,4-dihydro-3(2*H*)-quinazolinyl)phényl-carbamate de *tert*-butyle, 1-[2-benzyl-3-(1*H*-imidazol-1-yl)propyl]-3-(3-bromophényl)-2,4(1*H*,3*H*)-quinazolinedione, bis trifluoroacétate de 1-[2-benzyl-3-(1-méthyl-1*H*-imidazol-5-yl) propyl]-3-phényl-2,4(1*H*,3*H*)-quinazolinedione, 3-(3-bromophényl)-1-{3-[1-(4-cyanobenzyl)-1*H*-imidazol-5-yl]propyl}-2,4(1*H*,3*H*)-quinazolinedione.

La présente invention concerne également le procédé de préparation des composés de formule (I) caractérisé en ce que l'on utilise comme produit de départ un composé de formule (II): 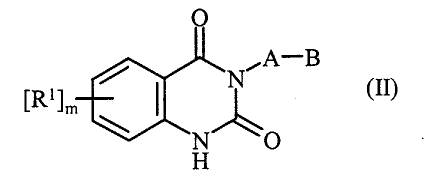 dans laquelle R¹, A, B et m sont tels que définis dans la formule (I) qui se condense avec le dérivé alcoolique de formule (III) : 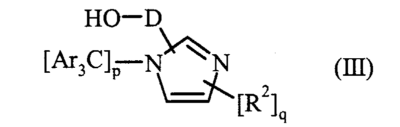 dans laquelle Ar représente un groupement phényle éventuellement substitué, q est un entier compris inclusivement entre 0 et 2, R² et D sont tels que définis dans la formule (I) et p est un entier compris inclusivement entre 0 et 1, pour conduire, en présence de dialkylazodicarboxylate et de triarylphosphine, au composé de formule (IV) : 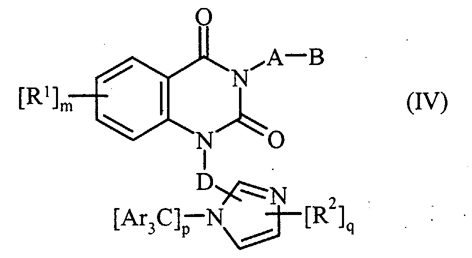 dans laquelle Ar, R¹, R², A, B, D, m, p et q sont tels que définis précédemment, qui est soumis à l'action d'agent de thionation de fonctions cétones, pour conduire au composé de formule (V) : 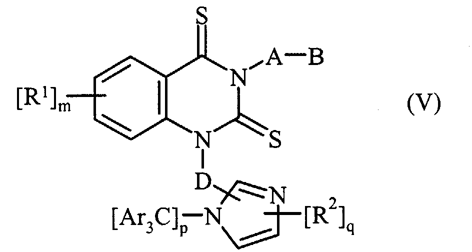 dans laquelle Ar, R¹, R², A, B, D, m, p et q sont tels que définis précédemment,
les composés de formules (IV) et (V) sont représentés par la formule (VI) : 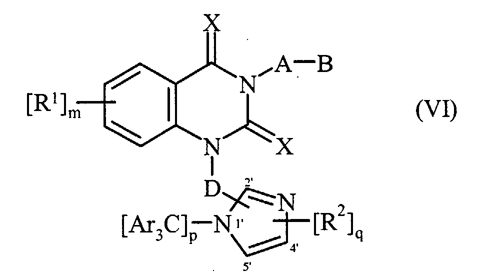 dans laquelle Ar, R¹, R², A, B, D, m, p, q sont tels que définis précédemment et X est tel que défini dans la formule (I),
* composé de formule (VI), lorsque D est relié au groupement imidazolyle par l'atome d'azote N_{1'} (où p vaut 0) correspond au composé de formule (I/a), cas particulier des composés de formule (I): 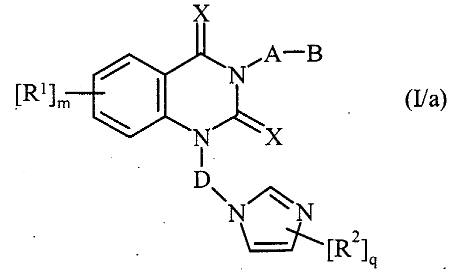 dans laquelle R¹, R², A, B, D, X, m et q sont tels que définis précédemment, qui peut être soumis au réactif suivant : Hal-R³ dans lequel Hal représente un atome d'halogène et R³ peut prendre toutes les valeurs de R² à l'exception du groupement aryle et hétéroaryle,
   pour conduire au composé cationique (I/b), cas particulier des composés de formule (I): 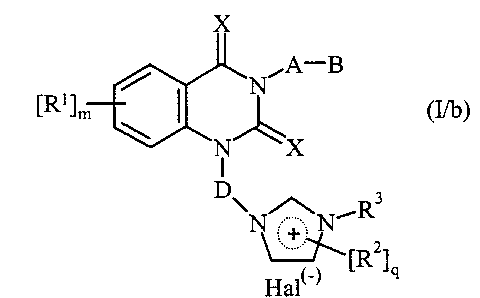 dans laquelle R¹, R², R³, A, B, D, X, m, q sont tels que définis précédemment et Hal⁽⁻⁾ représente l'anion correspondant à l'atome d'halogène Hal,
* composé de formule (VI), lorsque D est relié au groupement imidazolyle par les atomes de carbone C_{2'}, C_{4'} et C_{5'} (où p vaut 1) correspond au composé de formule (VII) : 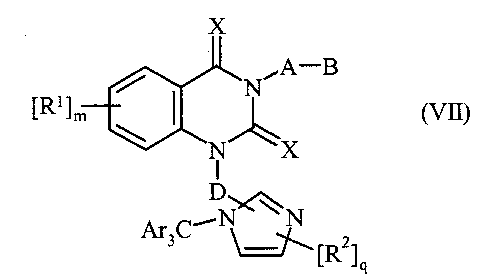 dans laquelle Ar, R¹, R², A, B, D, X, m et q sont tels que définis précédemment, qui peut :
   → **soit** être soumis à l'action d'un acide tel que l'acide chlorhydrique par exemple pour obtenir le composé déprotégé (I/c), cas particulier des composés de formule (I) : 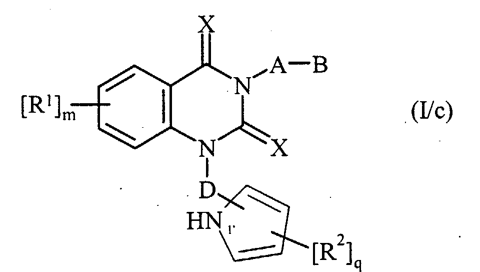
   dans laquelle R¹, R², A, B, D, X, m et q sont tels que définis précédemment,
   dont l'atome d'azote N₁, du groupement imidazolyle peut être substitué, en présence éventuellement d'un catalyseur approprié, par le réactif suivant : Hal-R⁴, dans lequel Hal est tel que défini précédemment et R⁴ peut prendre toutes les valeurs de R² à l'exception de l'atome d'hydrogène, des groupements aryle et hétéroaryle, pour conduire en milieu alcalin au composé de formule (I/d), cas particulier des composés de formule (I): 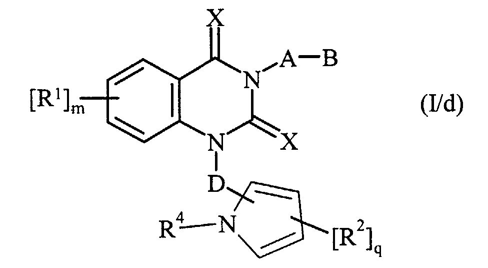 dans laquelle R¹, R², R⁴, A, B, D, X, m et q sont tels que définis précédemment,
   → **soit** réagir avec l'agent alkylant suivant : Hal-R⁵, dans lequel Hal représente un atome d'halogène et R⁵ peut prendre toutes les valeurs de R² à l'exception de l'atome d'hydrogène, des groupements aryle et hétéroaryle, pour conduire au composé cationique de formule (VIII) : 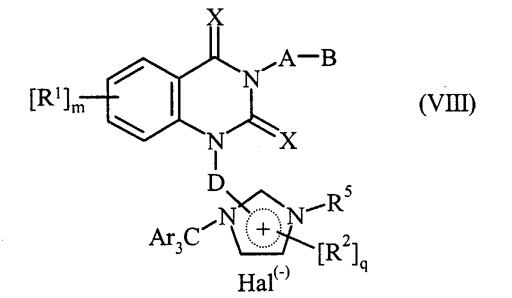
   dans laquelle Ar, R¹, R², R⁵, A, B, D, X, m, Hal⁽⁻⁾ et q sont tels que définis précédemment,
   qui est déprotégé, en chauffant dans le méthanol, pour conduire au composé de formule (I/e), cas particulier des composés de formule (I): 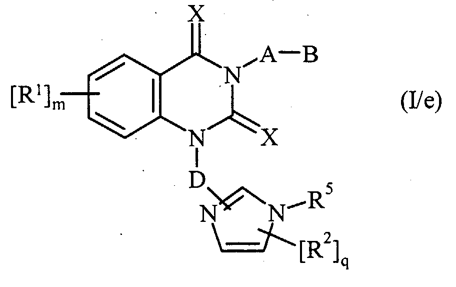 dans laquelle R¹, R², R⁵, A, B, D, X, m et q sont tels que définis précédemment,
   l'ensemble des composés de formule (I/a) à (I/e) formant l'ensemble des composés de formule (I),
   - qui peuvent être, le cas échéant, purifiés selon une technique classique de purification,
   - dont les isomères (diastéréoisomères, énantiomères et isomères géométriques) sont éventuellement séparés par une technique classique de séparation,
   - dont les intermédiaires peuvent être le cas échéant, protégés et déprotégés au cours de la synthèse pour faciliter l'accès aux produits recherchés,
   - que l'on transforme, si on le souhaite, en leurs sels d'addition à un acide ou à une base pharmaceutiquement acceptable.

La présente invention a également pour objet les compositions pharmaceutiques renfermant comme principe actif au moins un composé de formule (I), en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques et spectrométriques usuelles.

Les produits de départ utilisés sont des produits connus ou préparés selon des modes opératoires connus.

### PREPARATION 1 : 3-(3-Bromophényl)-2,4(1H,3H)-quinazolinedione

Un mélange de 10 g d'anhydride isatoïque de 6 g de 3-bromoaniline et de 20 g de carbonyldiimidazole est agité à 120 °C pendant 1 h. 30 min.. Après retour à température ambiante, 100 ml. d'eau sont ajoutés au résidu solide, les insolubles sont récupérés par filtration et lavés avec un minimum d'éthanol. Les 15 g de solide brut obtenus sont purifiés par chromatographie sur gel de silice (dichlorométhane/acétate d'éthyle : 95/5). 10 g du produit attendu sont obtenus.
Point de fusion : (Cap) >250 °C

| Microanalyses élémentaires : | | | | |
|---|---|---|---|---|
| | C | H | N | Br |
| % Calculé | 53,02 | 2,86 | 8,83 | 5,19 |
| % Trouvé | 52,73 | 2,84 | 8,60 | 4,90 |

### PREPARATION 2 : 4-(2,4-Dioxo-1,4-dihydro-3(2H)-quinazolinyl)phénylcarbamate de tert-butyle

Le produit est obtenu en procédant comme dans la Préparation 1 en remplaçant la 3-bromo aniline par le 4-anilinocarbamate de *tert*-butyle.

| Microanalyses élémentaires : | | | |
|---|---|---|---|
| | C | H | N |
| % Calculé | 64,58 | 5,42 | 11,89 |
| % Trouvé | 64,22 | 5,37 | 11,62 |

### PREPARATION 3 : 3-(Phénylsulfonyl)-2,4(1H,3H)-quinazolinedione

Le produit est obtenu en procédant selon le mode opératoire décrit dans la demande de brevet WO 00/10982.

### PREPARATION 4 : 3-(3-Thiènylsulfonyl)-2,4(1H,3H)-quinazolinedione

Le produit est obtenu en procédant comme dans la Préparation 3 mais à partir du 3-thiophènesulfonamide.

| Microanalyses élémentaires : | | | | |
|---|---|---|---|---|
| | C | H | N | S |
| % Calculé | 46,74 | 2,62 | 9,09 | 20,80 |
| % Trouvé | 47,35 | 2,89 | 9,53 | 21,22 |

### PREPARATION 5 : 3-Méthyl-2,4(1H,3H)-quinazolinedione

A une solution de 10 g de 2-amino-*N*-méthylbenzamide dans 500 ml. de dichlorométhane est ajoutée une solution de 6,6 g de triphosgène dans 100 ml. de dichlorométhane en 30 min.. Le milieu réactionnel est agité 1 h. à 60°C, le solvant est évaporé. Les 10,5 g de produit brut obtenus sont purifiés par chromatographie sur gel de silice (toluène/méthanol /NH₃ : 95/5/0,1). 2,2 g de produit sont obtenus.
Point de fusion : (Cap) 205-209 °C

| Microanalyses élémentaires : | | | |
|---|---|---|---|
| | C | H | N |
| % Calculé | 61,36 | 4,58 | 15,90 |
| % Trouvé | 61,66 | 4,38 | 15,00 |

### PREPARATION 6 : 3-(1H-Imidazol-1-yl)-1-propanol

A une suspension de 68 g d'imidazole et de 43 g de soude dans 100 ml. de butanol, 100 ml de bromopropanol sont ajoutés goutte à goutte en 1 h. Le milieu réactionnel est agité à 140°C pendant ½ heure. Après retour à température ambiante, les insolubles sont éliminés par filtration et lavés avec de l'éthanol. Après évaporation du solvant, l'huile obtenue est purifiée par chromatographie sur gel de silice (dichlorométhane/méthanol/NH₃ : 95/5/0,1). 95 g de produit sous forme d'huile sont obtenus.

| Microanalyses élémentaires : | | | |
|---|---|---|---|
| | C | H | N |
| % Calculé | 57,12 | 7,94 | 22,20 |
| % Trouvé | 57,35 | 7,42 | 21,80 |

### PREPARATION 7 : 3-(1H-Imidazol-1-yl)-2-phényl-1-propanol

### Stade a : 3-(1H-Imidazol-1-yl)-2-phénylpropanoate d'éthyle

A une solution de 10 g d'atropate d'éthyle [*Helvetica Chimica Acta*, **69,** 2048 (1986)] dans 100 ml. d'éthanol, 10 g d'imidazole sont ajoutés. Le milieu réactionnel est agité 24 h. à température ambiante et le solvant est évaporé sous pression réduite. Les 20 g d'huile obtenus sont purifiés par chromatographie sur gel de silice (dichlorométhane/méthanol/NH₃: 95/5/0,1). 11 g de produit sous forme d'huile sont obtenus.

| Microanalyses élémentaires : | | | |
|---|---|---|---|
| | C | H | N |
| % Calculé | 68,83 | 6,60 | 11,47 |
| % Trouvé | 68,38 | 6,76 | 11,18 |

### Stade b : 3-(1H-Imidazol-1-yl)-2-phényl-1-propanol

2,2 g d'hydrure de lithiumaluminium (LiAlH₄) sont ajoutés en 30 min. sous atmosphère inerte à une solution de 11 g de 3-(lH-imidazol-1-yl)-2-phénylpropanoate d'éthyle dans 300 ml. de THF à 0°C. Le milieu réactionnel est ensuite agité pendant 16 h. à température ambiante. Après hydrolyse par du sulfate de sodium (Na₂SO₄) humide, les insolubles sont éliminés par filtration. Le solvant est évaporé sous pression réduite, le résidu est repris avec du dichlorométhane, lavé à l'eau et la phase organique est séchée sur sulfate de magnésium. 6 g de produit sont obtenus.

| Microanalyses élémentaires : | | | |
|---|---|---|---|
| | C | H | N |
| % Calculé | 71,76 | 6,48 | 13,85 |
| % Trouvé | 72,67 | 7,00 | 12,49 |

### PREPARATION 8 : 4-[2-Hydroxy-1-(1H-imidazol-1-ylméthyl)éthyl]benzonitrile

Le 2-(4-bromophényl)-3-imidazol-1-yl-1-propanol est préparé selon les mêmes conditions opératoires que dans la Préparation 7.

A une solution de 5,5 g de 2-(4-bromophényl)-3-imidazol-1-yl-1-propanol dans 30 ml. de DMF dégazé sont ajoutés 5 g de cyanure de zinc (ZnCN₂) et 1,5 g de tétrakis(triphénylphosphine) (Pd(PPh₃)₄). Le milieu réactionnel est agité 15 min. au microonde à la puissance de 300 W. Les insolubles sont éliminés par filtration. Les 7,5 g de produit brut récupérés après évaporation du solvant sont purifiés par chromatographie sur gel de silice (dichlorométhane/méthanol/NH₃ : 95/5/0,1). 4,2 g de produit sont obtenus.
Point de fusion : (Cap) 140-143 °C

| Microanalyses élémentaires : | | | |
|---|---|---|---|
| | C | H | N |
| % Calculé | 68,71 | 5,77 | 18,49 |
| % Trouvé | 67,95 | 5,58 | 18,10 |

### PREPARATION 9 : 2-Benzyl-3-(1H-imidazol-1-yl)-1-propanol

Le produit est obtenu en procédant comme dans la Préparation 7 en partant du 2-benzylacrylate d'éthyle [*Synthetic Communication*, **18,** 11, 1213 (1988)] à la place de l'atropate d'éthyle. 6,5 g de produit sont obtenus sous forme d'huile.

| Microanalyses élémentaires : | | | |
|---|---|---|---|
| | C | H | N |
| % Calculé | 72,19 | 7,16 | 12,95 |
| % Trouvé | 71,85 | 7,01 | 12,65 |

### PREPARATION 10 : 3-(1H-Imidazol-1-yl)-1-phényl-1-propanol

### Stade a : 3-(1H-Imidazol-1-yl)-1-phényl-1-propanone

Une suspension de 1,7 g de 3-chloropropiophénone, de 0,1 g de l'iodure de potassium (KI) et de 2 g d'imidazole est chauffée à 150 °C pendant 16h.. Après retour à température ambiante, le produit est purifié par chromatographie sur gel de silice (dichlorométhane/éthanol : 98/2). 0,9 g de produit est obtenu.

| Microanalyses élémentaires : | | | |
|---|---|---|---|
| | C | H | N |
| % Calculé | 71,98 | 6,04 | 13,99 |
| % Trouvé | 71,97 | 6,06 | 13,86 |

### Stade b : 3-(1H-Imidazol-1-yl)-1-phényl-1-propanol

A 4 g de composé préparé au stade précédent en solution dans 60 ml. de méthanol, 2 g de de borohydrure de sodium sont ajoutés en 30 min.. Le milieu réactionnel est agité 2 h. à température ambiante. Après évaporation du solvant, le produit est purifié par chromatographie sur gel de silice (dichlorométhane/méthanol : 95/5). 1,6 g de produit est obtenu.
Point de fusion : (Cap) 108-110 °C

| Microanalyses élémentaires : | | | |
|---|---|---|---|
| | C | H | N |
| % Calculé | 71,26 | 6,98 | 13,85 |
| % Trouvé | 70,55 | 6,86 | 13,48 |

### PREPARATION 11 : 4-[1-Hydroxy-3-(1H-imidazol-1-yl)propyl]benzonitrile

Le 1-(4-bromophényl)-3-imidazol-1-yl-1-propanol est synthétisé comme dans la préparation 10 en partant de la 1-[(4-bromophényl)-3-imidazol-1-yl]phényl-1-propanone. La substitution du brome par un groupement cyano est réalisée dans les mêmes conditions opératoires que dans la préparation 8. 4,5 g de produit sont obtenus.
Point de fusion : (Cap) 94-97 °C

| Microanalyses élémentaires : | | | |
|---|---|---|---|
| | C | H | N |
| % Calculé | 68,71 | 5,77 | 18,49 |
| % Trouvé | 67,83 | 5,45 | 18,02 |

### PREPARATION 12 : 3-(1H-Imidazol-1-yl)-3-phényl-1-propanol

Une suspension de 1,6 g de 3-(1*H*-imidazol-1-yl)-3-phénylacrylate d'éthyle [*Tetrahedron Letters,* **37,** 40, 7249 (1996) et de 0,6 g de palladium sur graphite (Pd/C) à 5 % dans 10 ml d'éthanol est agitée sous 60 psi d'hydrogène, maintenue pendant 24 h à température ambiante. Le catalyseur est éliminé par filtration. Après évaporation du solvant 1,1 g de 3-(1*H*-imidazol-1-yl)-3-phénylpropanoate d'éthyle est obtenu. Ce dernier est réduit dans les mêmes conditions opératoires que celles utilisées dans la Préparation 7. 0,4 g de produit est obtenu.
Point de fusion : (Cap) 63-66 °C

| Microanalyses élémentaires : | | | |
|---|---|---|---|
| | C | H | N |
| % Calculé | 71,26 | 6,98 | 13,85 |
| % Trouvé | 70,82 | 6,51 | 13,63 |

### PREPARATION 13 : 3-(1-Trityl-1H-imidazol-2-yl)-1-propanol

### Stade a : 3-(1H-Imidazol-2-yl)acrylate d'éthyle

Une suspension de 10,9 g de 1*H*-imidazole-2-carbaldéhyde, 47 g de K₂CO₃ et 28 ml. de triéthoxyphosphonoacétate d'éthyle dans 550 ml. d'éthanol est agitée à 70 °C pendant 1 h..

Les insolubles sont éliminés par filtration, le solvant est évaporé sous pression réduite.

L'huile obtenue est purifiée par chromatographie sur gel de silice (dichlorométhane/méthanol: 95/5). 12,7 g de produit sont obtenus.

| Microanalyses élémentaires : | | | |
|---|---|---|---|
| | C | H | N |
| % Calculé | 57,82 | 6,07 | 16,86 |
| % Trouvé | 57,61 | 6,06 | 16,87 |

### Stade b : 3-(1H-Imidazol-2-yl)propanoate d'éthyle

Une suspension de 12,5 g de composé préparé au stade précédent et de 1,5 g de Pd/C (10%) dans 500 ml d'éthanol est agitée sous 60 psi d'hydrogène à température ambiante pendant 16 h.. Le catalyseur est éliminé par filtration, le solvant est évaporé sous pression réduite. 12,5 g de produit sont obtenus.
Point de fusion : (Cap) 107-109 °C

| Microanalyses élémentaires : | | | |
|---|---|---|---|
| | C | H | N |
| % Calculé | 57,13 | 7,19 | 16,66 |
| % Trouvé | 57,06 | 7,18 | 16,55 |

### Stade c : 3-(1-Trityl-1H-imidazol-2-yl)propanoate d'éthyle

Une solution de 8,4 g de composé préparé au stade précédant, de 15 ml. de triéthylamine, de 15 g de chlorure de trityle dans 50 ml de diméthylformamide (DMF) est agitée 16 h. à température ambiante. 150 ml d'eau sont ajoutés au milieu réactionnel. 21 g de produit sont récupérés après filtration.
Point de fusion : (Cap) 222-225 °C

| Microanalyses élémentaires : | | | |
|---|---|---|---|
| | C | H | N |
| % Calculé | 79,00 | 6,38 | 6,82 |
| % Trouvé | 79,06 | 6,42 | 6,44 |

### Stade d : 3-(1-Trityl-1H-imidazol-2-yl)-1-propanol

L'ester préparé au stade précédent est réduit avec l'hydrure de lithium aluminium (LiAlH₄) dans les mêmes conditions opératoires que celles utilisées dans la Préparation 7.
Point de fusion : (Cap) 138-140 °C

| Microanalyses élémentaires : | | | |
|---|---|---|---|
| | C | H | N |
| % Calculé | 81,49 | 6,56 | 7,60 |
| % Trouvé | 80,85 | 6,23 | 7,41 |

### PREPARATION 14 : 3-(1-Trityl-1H-imidazol-4-yl)-1-propanol

Le composé est synthétisé à partir du 3-(1*H*-imidazol-4-yl)acrylate de méthyle selon les modes opératoires des Stades a-d décrits précédemment dans la Préparation 13.

| Microanalyses élémentaires : | | | |
|---|---|---|---|
| | C | H | N |
| % Calculé | 81,49 | 6,56 | 7,60 |
| % Trouvé | 81,26 | 6,59 | 6,55 |

### PREPARATION 15 : 3-(4-Chlorophényl)-2,4(1H,3H)-quinazolinedione

Le produit est obtenu en procédant comme dans la Préparation 1 en remplaçant la 3-bromo aniline par la 4-chloroaniline.

### PREPARATION 16 : 3-(2-Méthoxyphényl)-2,4(1H,3H)-quinazolinedione

Le produit est obtenu en procédant comme dans la Préparation 1 en remplaçant la 3-bromo aniline par la 2-méthoxyaniline.

### PREPARATION 17 : 3-(2-Fluorophényl)-2,4(1H,3H)-quinazolinedione

Le produit est obtenu en procédant comme dans la Préparation 1 en remplaçant la 3-bromo aniline par la 2-fluoroaniline.

### PREPARATION 18 : 3-(2-Chlorophényl)-2,4(1H,3H)-quinazolinedione

Le produit est obtenu en procédant comme dans la Préparation 1 en remplaçant la 3-bromo aniline par la 2-chloroaniline.

### PREPARATION 19 : 3-(2-Bromophényl)-2,4(1H,3H)-quinazolinedione

Le produit est obtenu en procédant comme dans la Préparation 1 en remplaçant la 3-bromo aniline par la 2-bromoaniline.

### PREPARATION 20 : 3-(2-Trifluorométhylphényl)-2,4(1H,3H)-quinazolinedione

Le produit est obtenu en procédant comme dans la Préparation 1 en remplaçant la 3-bromo aniline par la 2-trifluorométhylaniline.

### PREPARATION 21 : 3-(2-Nitrophényl)-2,4(1H,3H)-quinazolinedione

Le produit est obtenu en procédant comme dans la Préparation 1 en remplaçant la 3-bromo aniline par la 2-nitroaniline.

### PREPARATION 22 : 3-(2-Anilinophényl)-2,4(1H,3H)-quinazolinedione

Le produit est obtenu en procédant comme dans la Préparation 1 en remplaçant la 3-bromo aniline par la 2-anilinoaniline.

### PREPARATION 23 : 3-(2-Benzylphényl)-2,4(1H,3H)-quinazolinedione

Le produit est obtenu en procédant comme dans la Préparation 1 en remplaçant la 3-bromo aniline par la 2-benzylaniline.

### PREPARATION 24 : 3-(3-Fluorophényl)-2,4(1H,3H)-quinazolinedione

Le produit est obtenu en procédant comme dans la Préparation 1 en remplaçant la 3-bromo aniline par la 3-fluoroaniline.

### PREPARATION 25 : 3-(3-Chlorophényl)-2,4(1H,3H)-quinazolinedione

Le produit est obtenu en procédant comme dans la Préparation 1 en remplaçant la 3-bromo aniline par la 3-chloroaniline.

### PREPARATION 26 : 3-(3-Trifluorométhylphényl)-2,4(1H,3H)-quinazolinedione

Le produit est obtenu en procédant comme dans la Préparation 1 en remplaçant la 3-bromo aniline par la 3-trifluorométhylaniline.

### PREPARATION 27 : 3-(3-Nitrophényl)-2,4(1H,3H)-quinazolinedione

Le produit est obtenu en procédant comme dans la Préparation 1 en remplaçant la 3-bromo aniline par la 3-nitroaniline.

### PREPARATION 28 : 3-(3-Cyanophényl)-2,4(1H,3H)-quinazolinedione

Le produit est obtenu en procédant comme dans la Préparation 1 en remplaçant la 3-bromo aniline par la 3-cyanoaniline.

### PREPARATION 29 : 3-(3-Méthoxyphényl)-2,4(1H,3H)-quinazolinedione

Le produit est obtenu en procédant comme dans la Préparation 1 en remplaçant la 3-bromo aniline par la 3-méthoxyaniline.

### PREPARATION 30 : 3-(3-Diméthylaminophényl)-2,4(1H,3H)-quinazolinedione

Le produit est obtenu en procédant comme dans la Préparation 1 en remplaçant la 3-bromo aniline par la 3-diméthylaminoaniline.

### PREPARATION 31 : 3-(3,5-dichlorophényl)-2,4(1H,3H)-quinazolinedione

Le produit est obtenu en procédant comme dans la Préparation 1 en remplaçant la 3-bromo aniline par la 3,5-dichloroaniline.

### PREPARATION 32 : 3-[3,5-Bis(trifluorométhyl)phényl]-2,4(1H,3H)-quinazolinedione

Le produit est obtenu en procédant comme dans la Préparation 1 en remplaçant la 3-bromo aniline par la 3,5-di(trifluorométhyl)aniline.

### PREPARATION 33 : 3-(2,3-Diméthylphényl)-2,4(1H,3H)-quinazolinedione

Le produit est obtenu en procédant comme dans la Préparation 1 en remplaçant la 3-bromo aniline par la 2,3-diméthylaniline.

### PREPARATION 34 : 3-(4-Fluorophényl)-2,4(1H,3H)-quinazolinedione

Le produit est obtenu en procédant comme dans la Préparation 1 en remplaçant la 3-bromo aniline par la 4-fluoroaniline.

### PREPARATION 35 : 3-(4-Bromophényl)-2,4(1H,3H)-quinazolinedione

Le produit est obtenu en procédant comme dans la Préparation 1 en remplaçant la 3-bromo aniline par la 4-bromoaniline.

### PREPARATION 36 : 3-(4-Trifluorométhylphényl)-2,4(1H,3H)-quinazolinedione

Le produit est obtenu en procédant comme dans la Préparation 1 en remplaçant la 3-bromo aniline par la 4-trifluorométhylaniline.

### PREPARATION 37 : 3-(4-Nitrophényl)-2,4(1H,3H)-quinazolinedione

Le produit est obtenu en procédant comme dans la Préparation 1 en remplaçant la 3-bromo aniline par la 4-nitroaniline.

### PREPARATION 38 : 3-(4-Cyanophényl)-2,4(1H,3H)-quinazolinedione

Le produit est obtenu en procédant comme dans la Préparation 1 en remplaçant la 3-bromo aniline par la 4-cyanoaniline.

### PREPARATION 39 : 3-(4-Méthylphényl)-2,4(1H,3H)-quinazolinedione

Le produit est obtenu en procédant comme dans la Préparation 1 en remplaçant la 3-bromo aniline par la 4-méthylaniline.

### PREPARATION 40 : 3-(4-Méthoxyphényl)-2,4(1H,3H)-quinazolinedione

Le produit est obtenu en procédant comme dans la Préparation 1 en remplaçant la 3-bromo aniline par la 4-méthoxyaniline.

### PREPARATION 41 : 3-(4-Diméthylaminophényl)-2,4(1H,3H)-quinazolinedione

Le produit est obtenu en procédant comme dans la Préparation 1 en remplaçant la 3-bromo aniline par la 4-diméthylaminoaniline.

### PREPARATION 42 : 3-(4-Anilinophényl)-2,4(1H,3H)-quinazolinedione

Le produit est obtenu en procédant comme dans la Préparation 1 en remplaçant la 3-bromoaniline par la 4-anilinophénylamine .

### PREPARATION 43 : 3-(2-Pyridyl)-2,4(1H,3H)-quinazolinedione

Le produit est obtenu en procédant comme dans la Préparation 1 en remplaçant la 3-bromo aniline par la 2-aminopyridine.

### PREPARATION 44 : 3-(3-Pyridyl)-2,4(1H,3H)-quinazolinedione

Le produit est obtenu en procédant comme dans la Préparation 1 en remplaçant la 3-bromo aniline par la 3-aminopyridine.

### PREPARATION 45 : 3-(4-Pyridyl)-2,4(1H,3H)-quinazolinedione

Le produit est obtenu en procédant comme dans la Préparation 1 en remplaçant la 3-bromo aniline par la 4-aminopyridine.

### PREPARATION 46 : 3-Cyclohexyl-2,4(1H,3H)-quinazolinedione

Le produit est obtenu en procédant comme dans la Préparation 1 en remplaçant la 3-bromo aniline par la cyclohexanamine.

### PREPARATION 47 : 3-Bicyclo[2.2.1]hept-2-yl-2,4(1H,3H)-quinazolinedione

Le produit est obtenu en procédant comme dans la Préparation 1 en remplaçant la 3-bromo aniline par la bicyclo[2.2.1]hept-2-ylamine.

### PREPARATION 48 : 3-(1,3-Benzodioxol-5-yl)-2,4(1H,3H)-quinazolinedione

Le produit est obtenu en procédant comme dans la Préparation 1 en remplaçant la 3-bromo aniline par la 1,3-benzodioxol-5-ylamine.

### PREPARATION 49 : 3-(1-Naphthyl)-2,4(1H,3H)-quinazolinedione

Le produit est obtenu en procédant comme dans la Préparation 1 en remplaçant la 3-bromo aniline par la 1-naphtylamine.

### PREPARATION 50 : 3-Benzyl-2,4(1H,3H)-quinazolinedione

Le produit est obtenu en procédant comme dans la Préparation 1 en remplaçant la 3-bromo aniline par la benzylamine.

### PREPARATION 51 : 3-(2-Phénéthyl)-2,4(1H,3H)-quinazolinedione

Le produit est obtenu en procédant comme dans la Préparation 1 en remplaçant la 3-bromo aniline par la 2-phénéthylamine.

### PREPARATION 52 : 3-(2-Phénylpropyl)-2,4(1H,3H)-quinazolinedione

Le produit est obtenu en procédant comme dans la Préparation 1 en remplaçant la 3-bromo aniline par la 2-phénylpropylamine.

### PREPARATION 53 : 3-(2-Phénylbutyl)-2,4(1H,3H)-quinazolinedione

Le produit est obtenu en procédant comme dans la Préparation 1 en remplaçant la 3-bromo aniline par la 2-phénylbutylamine.

### PREPARATION 54 : 3-(2-Pyridylméthyl)-2,4(1H,3H)-quinazolinedione

Le produit est obtenu en procédant comme dans la Préparation 1 en remplaçant la 3-bromo aniline par la 2-pyridylméthylamine.

### PREPARATION 55 : 3-(3-Pyridylméthyl)-2,4(1H,3H)-quinazolinedione

Le produit est obtenu en procédant comme dans la Préparation 1 en remplaçant la 3-bromo aniline par la 3-pyridylméthylamine.

### PREPARATION 56 : 3-(Cyclopropylméthyl)-2,4(1H,3H)-quinazolinedione

Le produit est obtenu en procédant comme dans la Préparation 1 en remplaçant la 3-bromo aniline par la cyclopropylméthylamine.

### PREPARATION 57 : 3-(1-Adamantylméthyl)-2,4(1H,3H)-quinazolinedione

Le produit est obtenu en procédant comme dans la Préparation 1 en remplaçant la 3-bromo aniline par la 1-adamantylméthylamine.

### PREPARATION 58 : 3-(2-Oxo-2-phénéthyl)-2,4(1H,3H)-quinazolinedione

Le produit est obtenu en procédant comme dans la Préparation 1 en remplaçant la 3-bromo aniline par la 2-oxophénéthylamine.

### PREPARATION 59 : 3-(3,4-Dichlorophényl)-2,4(1H,3H)-quinazolinedione

Le produit est obtenu en procédant comme dans la Préparation 1 en remplaçant la 3-bromo aniline par la 3,4-dichloroaniline.

### PREPARATION 60 : 3-(2-Cyanophényl)-2,4(1H,3H)-quinazolinedione

Le produit est obtenu en procédant comme dans la Préparation 1 en remplaçant la 3-bromo aniline par la 2-cyanoaniline.

### PREPARATION 61 : 2-(4-Bromobenzyl)-3-(1H-imidazol-1-yl)-1-propanol

### Stade a : 2-(4-Bromobenzyl)-3-(1H-imidazol-1-yl)propanoate d'éthyle

Une suspension de 60 ml d'éthylbenzoylacétate, 100 g de 4-bromobenzylbromide, 84 g de carbonate de potassium et 16 g d'iodure de sodium dans 200 ml de THF est agitée 4 heures à 60°C. 56 g de carbonate de potassium, 20 g de paraformaldéhyde et 400 ml de THF sont ajoutés au milieu réactionnel, le mélange est agité 20 heures à 60°C. Le solvant est évaporé. Le produit est extrait avec de l'éther. Les 125 g d'huile sont repris par 1 litre d'éthanol, 125 g d'imidazole sont ajoutés à la solution. Le mélange est agité 20 heures à 70°C. Le solvant est évaporé sous pression réduite. Le produit obtenu est purifié par chromatographie sur gel de silice (dichlorométhane/méthanol-NH₃ : 95/5). 208 g de produit sont obtenus.

### Stade b : 2-(4-Bromobenzyl)-3-(1H-imidazol-1-yl)-1-propanol

6,5 g d'hydrure de lithiumaluminium (LiAlH₄) sont ajoutés en 30 minutes à une solution du composé du stade a précédent dans 800 ml d'éther. Le milieu réactionnel est agité 2 heures à 25°C. 50 g de sulfate de sodium humide sont ajoutés au milieu réactionnel en 20 minutes. Les insolubles sont éliminés par filtration. Le solvant est évaporé sous pression réduite. 23 g de produit sont obtenus par cristallisation dans l'éther.

### PREPARATION 62 : 2-(4-Cyanobenzyl)-3-(1H-imidazol-1-yl)-1-propanol

Une suspension de 5 g du composé de la préparation 61, 4,5 g de Zn(CN)₂, 1,2 g de Pd(PPh₃)₄ dans 25 ml de diméthylformamide est agitée 15 minutes au micro-ondes. Les insolubles sont éliminés par filtration. Le produit est purifié par chromatographie sur gel de silice (dichlorométhane/méthanol: 95/5). 4,5 g de produit sont obtenus.

### EXEMPLE 1 : Fumarate de 1-[3-(1H-imidazol-1-yl)propyl]-3-phényl-2,4(1H,3H)-quinazolinedione

A une suspension de 2 g du produit commercial 3-phényl-2,4(1*H*,3*H*)-quinazolinedione, 1,2 g du produit de la Préparation 6 et 5 g de triphénylphosphine sur résine dans 100 ml de tétrahydrofurane (THF), est ajoutée goutte à goutte une solution de 1,5 ml de diéthylazodicarboxylate dans 10 ml de THF. Le milieu réactionnel est agité pendant 16 h., les insolubles sont éliminés par filtration. Les 5 g de produit récupérés après évaporation du solvant sont purifiés par chromatographie sur gel de silice (foluène/méthanol : 95/5), le produit obtenu est transformé en son sel de fumarate par addition d'acide fumarique dans l'éthanol.
Point de fusion : (Cap) 174-176 °C

| Microanalyses élémentaires : | | | |
|---|---|---|---|
| | C | H | N |
| % Calculé | 62,33 | 4,79 | 12,11 |
| % Trouvé | 61,60 | 4,81 | 11,63 |

### EXEMPLE 2 : 4-(1-[2-Benzyl-3-(1H-imidazol-1-yl)propyl]-2,4-dioxo-1,4-dihydro-3(2H)-quinazolinyl)phénylcarbamate de tert-butyle

Le protocole expérimental est identique à celui de l'Exemple 1 en utilisant à la place de la 3-phényl-2,4(1*H*,3*H*)-quinazolinedione le composé de la Préparation 2 et à la place du produit de la Préparation 6 celui de la Préparation 9.

| Microanalyses élémentaires : | | | |
|---|---|---|---|
| | C | H | N |
| % Calculé | 69,67 | 6,03 | 12,70 |
| % Trouvé | 68,49 | 6,22 | 11,98 |

La séparation des énantiomères de l'Exemple 2 a été effectuée sur colonne chirale permettant ainsi d'obtenir les deux énantiomères (*R*) et (*S*) optiquement purs à plus de 99% :
Enantiomère 1 :
Point de fusion : (Cap) déc.>150 °C

| Microanalyses élémentaires : | | | |
|---|---|---|---|
| | C | H | N |
| % Calculé | 69,67 | 6,03 | 12,70 |
| % Trouvé | 68,56 | 5,89 | 12,19 |

Enantiomère 2 :
Point de fusion : (Cap) déc.>150 °C

| Microanalyses élémentaires : | | | |
|---|---|---|---|
| | C | H | N |
| % Calculé | 69,67 | 6,03 | 12,70 |
| % Trouvé | 68,38 | 6,09 | 12,33 |

### EXEMPLE 3 : 1-[2-Benzyl-3-(1H-imidazol-1-yl)propyl]-3-(3-bromophényl)-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 1 et à la place du produit de la Préparation 6 celui de la Préparation 9.

| Microanalyses élémentaires : | | | | |
|---|---|---|---|---|
| | C | H | N | Br |
| % Calculé | 62,92 | 4,50 | 10,87 | 15,50 |
| % Trouvé | 62,31 | 4,72 | 10,34 | 15,21 |

### EXEMPLE 4 : 1-[3-(1H-Imidazol-1-yl)-2-phénylpropyl]-3-(phénylsulfonyl)-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 3 et à la place du produit de la Préparation 6 celui de la Préparation 7.

| Microanalyses élémentaires : | | | | |
|---|---|---|---|---|
| | C | H | N | S |
| % Calculé | 64,18 | 4,56 | 11,52 | 6,59 |
| % Trouvé | 64,04 | 4,68 | 11,12 | 6,38 |

### EXEMPLE 5 : 1-[3-(1H-Imidazol-1-yl)-2-phénylpropyl]-3-(3-thiènylsulfonyl)-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 4 et à la place du produit de la Préparation 6 celui de la Préparation 7.

| Microanalyses élémentaires : | | | | |
|---|---|---|---|---|
| | C | H | N | S |
| % Calculé | 58,52 | 4,09 | 11,37 | 13,02 |
| % Trouvé | 58,44 | 4,47 | 10,96 | 13,18 |

### EXEMPLE 6 : 4-(1-(2-(4-Cyanophényl)-3-(1H-imidazol-1-yl)propyl]-2,4-dioxo-1,4-dihydro-3(2H)-quinazolinyl)phénylcarbamate de tert-butyle

Le protocole expérimental est identique à celui de l'Exemple 1 en utilisant à la place de la 3-phényl-2,4(1*H*,3*H*)-quinazolinedione le composé de la Préparation 2 et à la place du produit de la Préparation 6 celui de la Préparation 8.
Point de fusion : (Cap) déc.>220 °C

| Microanalyses élémentaires : | | | |
|---|---|---|---|
| | C | H | N |
| % Calculé | 68,31 | 5,37 | 14,94 |
| % Trouvé | 67,92 | 5,46 | 14,64 |

### EXEMPLE 7 : 4-(1-[1-(4-Cyanophényl)-3-(1H-imidazol-1-yl)propyl]-2,4-dioxo-1,4-dihydro-3(2H)-quinazolinyl)phénylcarbamate de tert-butyle

Le protocole expérimental est identique à celui de l'Exemple 1 en utilisant à la place de la 3-phényl-2,4(1*H*,3*H*)-quinazolinedione le composé de la Préparation 2 et à la place du produit de la Préparation 6 celui de la Préparation 11.

| Microanalyses élémentaires : | | | |
|---|---|---|---|
| | C | H | N |
| % Calculé | 68,31 | 5,37 | 14,94 |
| % Trouvé | 67,94 | 5,46 | 14,51 |

### EXEMPLE 8 : 1-[2-Benzyl-3-(1H-imidazol-1-yl)propyl]-3-phényl-2,4(1H,3H)-quinazolinedione

Le protocole expérimental est identique à celui de l'Exemple 1 en utilisant à la place du produit de la Préparation 6 celui de la Préparation 9.

| Microanalyses élémentaires : | | | |
|---|---|---|---|
| | C | H | N |
| % Calculé | 74,29 | 5,54 | 12,84 |
| % Trouvé | 73,49 | 6,30 | 10,89 |

La séparation des énantiomères de l'Exemple 8 a été effectuée sur colonne chirale permettant ainsi d'obtenir les deux énantiomères (*R*) et (*S*) optiquement purs à plus de 99% :

| Enantiomère 1 : | | | |
|---|---|---|---|
| | C | H | N |
| % Calculé | 74,29 | 5,54 | 12,84 |
| % Trouvé | 73,20 | 5,85 | 12,25 |

| Enantiomère 2 : | | | |
|---|---|---|---|
| | C | H | N |
| % Calculé | 74,29 | 5,54 | 12,84 |
| % Trouvé | 73,10 | 5,41 | 12,25 |

### EXEMPLE 9 : 4-(1-[3-(1H-Imidazol-1-yl)-2-phénylpropyl]-2,4-dioxo-1,4-dihydro-3(2H)-quinazolinyl)phénylcarbamate de tert-butyle

Le protocole expérimental est identique à celui de l'Exemple 1 en utilisant à la place de la 3-phényl-2,4(1*H*,3*H*)-quinazolinedione le composé de la Préparation 2 et à la place du produit de la Préparation 6 celui de la Préparation 7.
Point de fusion : (Cap) 178-191 °C

| Microanalyses élémentaires : | | | |
|---|---|---|---|
| | C | H | N |
| % Calculé | 69,26 | 5,81 | 13,03 |
| % Trouvé | 67,46 | 5,88 | 12,11 |

### EXEMPLE 10 : 3-(3-Bromophényl)-1-[3-(1H-imidazol-1-yl)-2-phénylpropyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 1 et à la place du produit de la Préparation 6 celui de la Préparation 7.

| Microanalyses élémentaires : | | | | |
|---|---|---|---|---|
| | C | H | N | Br |
| % Calculé | 62,29 | 4,22 | 11,17 | 15,94 |
| % Trouvé | 63,00 | 4,22 | 10,80 | 15,54 |

### EXEMPLE 11 : 4-[1-(3-(3-Bromophényl)-2,4-dioxo-3,4-dihydro-1(2H)-quinazolinyl)-3-(1H-imidazol-1-yl)propyl]benzonitrile

Le protocole expérimental est identique à celui de l'Exemple 1 en utilisant à la place de la 3-phényl-2,4(1*H*,3*H*)-quinazolinedione le composé de la Préparation 1 et à la place du produit de la Préparation 6 celui de la Préparation 11.

| Microanalyses élémentaires : | | | | |
|---|---|---|---|---|
| | C | H | N | Br |
| % Calculé | 61,61 | 3,83 | 13,30 | 15,18 |
| % Trouvé | 61,13 | 4,20 | 12,65 | 14,65 |

### EXEMPLE 12 : 1-[3-(1H-Imidazol-1-yl)-1-phénylpropyl]-3-phényl-2,4(1H,3H)-quinazolinedione

Le protocole expérimental est identique à celui de l'Exemple 1 en utilisant à la place du produit de la Préparation 6 celui de la Préparation 10.
Point de fusion : >230 °C

| Microanalyses élémentaires : | | | |
|---|---|---|---|
| | C | H | N |
| % Calculé | 73,92 | 5,25 | 13,26 |
| % Trouvé | 73,47 | 5,43 | 13,02 |

### EXEMPLE 13 : 1-[3-(1H-Imidazol-1-yl)-3-phénylpropyl]-3-phényl-2,4(1H,3H)-quinazolinedione

Le protocole expérimental est identique à celui de l'Exemple 1 en utilisant à la place du produit de la Préparation 6 celui de la Préparation 12.
Point de fusion : (Cap) 202-205 °C

| Microanalyses élémentaires : | | | |
|---|---|---|---|
| | C | H | N |
| % Calculé | 73,92 | 5,25 | 13,26 |
| % Trouvé | 73,69 | 5,36 | 13,29 |

### EXEMPLE 14 : 3-(3,4-Dichlorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 59.
Point de fusion : (Cap) 188-192 °C

| Microanalyses élémentaires : | | | | |
|---|---|---|---|---|
| | C | H | N | Cl |
| % Calculé | 57,85 | 3,88 | 13,49 | 17,07 |
| % Trouvé | 57,56 | 4,10 | 13,07 | 16,76 |

### EXEMPLE 15 : 1-[3-(1H-Imidazol-1-yl)-2-phénylpropyl]-3-phényl-2,4(1H,3H)-quinazolinedione

Le protocole expérimental est identique à celui de l'Exemple 1 en utilisant à la place du produit de la Préparation 6 celui de la Préparation 7.
Point de fusion : (Cap) 111-115 °C

| Microanalyses élémentaires : | | | |
|---|---|---|---|
| | C | H | N |
| % Calculé | 73,92 | 5,25 | 13,26 |
| % Trouvé | 73,52 | 5,49 | 12,39 |

La séparation des énantiomères de l'Exemple 15 a été effectuée sur colonne chirale permettant ainsi d'obtenir les deux énantiomères (*R*) et (*S*) optiquement purs à plus de 98% :
Enantiomère 1 :
Point de fusion : (Cap) 132-135 °C

| Microanalyses élémentaires : | | | |
|---|---|---|---|
| | C | H | N |
| % Calculé | 73,92 | 5,25 | 13,26 |
| % Trouvé | 73,31 | 5,16 | 13,00 |

Enantiomère 2 :
Point de fusion : (Cap) 133-136 °C

| Microanalyses élémentaires : | | | |
|---|---|---|---|
| | C | H | N |
| % Calculé | 73,92 | 5,25 | 13,26 |
| % Trouvé | 72,72 | 5,12 | 12,88 |

### EXEMPLE 16 : 1-[3-(1H-Imidazol-1-yl)propyl]-3-(2-méthoxyphényl)-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 16.

| Microanalyses élémentaires : | | | |
|---|---|---|---|
| | C | H | N |
| % Calculé | 67,01 | 5,36 | 14,88 |
| % Trouvé | 65,55 | 5,42 | 14,53 |

### EXEMPLE 17 : 4-(1-[3-(1H-Imidazol-1-yl)propyl]-2,4-dioxo-1,4-dihydro-3(2H)-quinazolinyl) phénylcarbamate de tert-butyle

Le protocole expérimental est identique à celui de l'Exemple 1 en utilisant à la place de la 3-phényl-2,4(1*H*,3*H*)-quinazolinedione le composé de la Préparation 2.
Point de fusion : (Cap) déc.>228 °C

| Microanalyses élémentaires : | | | |
|---|---|---|---|
| | C | H | N |
| % Calculé | 65,06 | 5,90 | 15,17 |
| % Trouvé | 63,04 | 5,89 | 14,51 |

### EXEMPLE 18 : 3-(3-Bromophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 1.

| Microanalyses élémentaires : | | | | |
|---|---|---|---|---|
| | C | H | N | Br |
| % Calculé | 56,48 | 4,03 | 13,17 | 18,79 |
| % Trouvé | 55,64 | 4,13 | 12,57 | 18,26 |

### EXEMPLE 19 : Fumarate de 1-[3-(1H-imidazol-1-yl)propyl]-3-méthyl-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 5.
Point de fusion : (Cap) 212-213 °C

| Microanalyses élémentaires : | | | |
|---|---|---|---|
| | C | H | N |
| % Calculé | 54,54 | 5,30 | 13,39 |
| % Trouvé | 53,92 | 5,17 | 13,02 |

### EXEMPLE 20 : 3-(2,3-Dimethylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 33.
Spectrométrie de masse : m /z = 375,1

### EXEMPLE 21 : Chlorhydrate de 1-[3-(1H-imidazol-4-yl)propyl]-3-phényl-2,4(1H,3H)-quinazolinedione

### Stade a : 3-Phényl-1-[3-(1-trityl-1H-imidazol-4-yl)propyl]-2,4(1H,3H)-quinazolinedione

Le composé a été synthétisé selon le même mode opératoire que celui de l'Exemple 1 à partir du produit commercial 3-phényl-2,4(1*H*,3*H*)-quinazolinedione et du produit de la Préparation 14.

### Stade b : 1-[3-(1H-Imidazol-4-yl)propyl]-3-phénol-2,4(1H,3H)-quinazolinedione

A une solution de 300 mg du composé préparé au stade précédent dans 3 ml de méthanol sont ajoutés 2 ml d'acide chlorhydrique 4N. Le milieu réactionnel est agité 16h. à 50 °C. Le solvant est évaporé sous pression réduite, le produit obtenu est cristallisé dans un mélange éthanol/éther et 180 mg de produit sont isolés.
Point de fusion : (Cap) 235-238 °C

| Microanalyses élémentaires : | | | | |
|---|---|---|---|---|
| | C | H | N | Cl |
| % Calculé | 62,74 | 5,01 | 14,64 | 9,26 |
| % Trouvé | 62,12 | 4,97 | 14,62 | 9,95 |

### EXEMPLE 22 : 1-[3-(1-Benzyl-1H-imidazol-4-yl)propyl]-3-phényl-2,4(1H,3H)-quinazolinedione

A une suspension de 150 mg du composé préparé dans l'Exemple 21, 100 mg de carbonate de potassium (K₂CO₃) dans 2 ml d'acétonitrile sont ajoutés 60 µl de bromure de benzyle. Le milieu réactionnel est agité pendant 16h. à température ambiante. Les insolubles sont éliminés par filtration, après évaporation du solvant, le produit est purifié par chromatographie sur gel de silice (toluène/méthanol : 97/3) pour isoler 50 mg de produit.

| Microanalyses élémentaires : | | | |
|---|---|---|---|
| | C | H | N |
| % Calculé | 74,29 | 5,54 | 12,84 |
| % Trouvé | 74,13 | 6,04 | 11,68 |

### EXEMPLE 22' : 1-[3-(1-Benzyl-1H-imidazol-5-yl)propyl]-3-phényl-2,4-(1H,3H)-quinazolinedione

### Stade a : 3-Phényl-1-[3-(1-trityl-1H-imidazol-4-yl)propyl]-2,4(1H,3H)-quinazolinedione

Le composé a été synthétisé selon le même mode opératoire que celui de l'Exemple 1 à partir du produit commercial 3-phényl-2,4(1*H*,3*H*)-quinazolinedione et du produit de la Préparation 14.

### Stade b : 1-[3-(1-Benzyl-1H-imidazol-5-yl)propyl]-3-phényl-2,4-(1H,3H)-quinazolinedione

A une solution du composé synthétisé au stade précédent (300 mg) dans 3 ml de méthyléthylcétone, sont ajoutés 80 µl de bromure de benzyle. Après avoir laissé le milieu réactionnel sous agitation pendant 2h à 55°C, le solvant est éliminé sous pression réduite. Le résidu solide est repris par 3 ml de méthanol et le mélange est chauffé 2h à 70°C.
Le solvant est ensuite évaporé et le solide brut est purifié par chromatographie sur gel de silice (dichlorométhane/méthanol: 95/5).

| Microanalyses élémentaires : | | | |
|---|---|---|---|
| | C | H | N |
| % Calculé | 74,29 | 5,54 | 12,84 |
| % Trouvé | 74,42 | 5,94 | 11,85 |

### EXEMPLE 23 : 1-[3-(1-Méthyl-1H-imidazol-4-yl)propyl]-3-phényl-2,4(1H,3H)-quinazolinedione

Le composé est préparé dans les mêmes conditions opératoires que pour l'Exemple 22 en utilisant de l'iodure de méthyle à la place du bromure de benzyle.

| Microanalyses élémentaires : | | | |
|---|---|---|---|
| | C | H | N |
| % Calculé | 69,98 | 5,59 | 15,55 |
| % Trouvé | 69,25 | 5,87 | 15,58 |

### EXEMPLE 24 : 1-[3-(1H-Imidazol-2-yl)propyl]-3-phényl-2,4(1H,3H)-quinazolinedione

### Stade a : 3-Phénvl-1-[3-(1-trityl-1H-imidazol-2-yl)propyl]-2,4(1H,3H)-quinazolinedione

Le composé a été synthétisé selon le même mode opératoire que celui de l'Exemple 1 à partir du produit commercial 3-phényl-2,4(1*H*,3*H*)-quinazolinedione et le produit de la Préparation 13.

### Stade b : 1-[3-(1H-Imidazol-2-yl)propyl]-3-phényl-2,4(1H,3H)-quinazolinedione

Le composé est préparé dans les mêmes conditions opératoires que pour l'Exemple 21 au Stade b en utilisant le composé préparé au stade précédent comme réactif de départ.
Point de fusion : (Cap) 230-232 °C

| Microanalyses élémentaires : | | | |
|---|---|---|---|
| | C | H | N |
| % Calculé | 69,35 | 5,24 | 16,17 |
| % Trouvé | 68,89 | 5,24 | 15,94 |

### EXEMPLE 25 : 1-[3-(1-Méthyl-1H-imidazol-2-yl)propyl]-3-phényl-2,4(1H,3H)-quinazolinedione

Le composé est préparé dans les mêmes conditions opératoires que pour l'Exemple 23 en utilisant comme produit départ le composé de l'Exemple 24.
Point de fusion : (Cap) 232-235 °C

| Microanalyses élémentaires : | | | |
|---|---|---|---|
| | C | H | N |
| % Calculé | 69,98 | 5,59 | 15,55 |
| % Trouvé | 70,09 | 5,60 | 15,34 |

### EXEMPLE 26 : 1-[3-(1-Benzyl-1H-imidazol-2-yl)propyl]-3-phényl-2,4(1H,3H)-quinazolinedione

Le composé est préparé dans les mêmes conditions opératoires que pour l'Exemple 22 en utilisant comme produit départ le composé de l'Exemple 24.
Point de fusion : (Cap) 182-185 °C

| Microanalyses élémentaires : | | | |
|---|---|---|---|
| | C | H | N |
| % Calculé | 74,29 | 5,54 | 12,84 |
| % Trouvé | 72,72 | 5,50 | 12,50 |

### EXEMPLE 27 : Iodure de 2-[3-(2,4-Dioxo-3-phényl-3,4-dihydro-1(2H)-quinazolinyl) propyl]-1,3-diméthyl-1H-imidazol-3-ium

Le composé est préparé dans les mêmes conditions opératoires que pour l'Exemple 25 en utilisant un large excès d'iodure de méthyle.
Point de fusion : (Cap) >240 °C

| Microanalyses élémentaires : | | | | |
|---|---|---|---|---|
| | C | H | N | I |
| % Calculé | 52,60 | 4,61 | 11,15 | 25,26 |
| % Trouvé | 50,53 | 4,55 | 10,53 | 22,98 |

### EXEMPLE 28 : 4-(1-[3-(1H-Imidazol-5-yl)propyl]-2,4-dioxo-1,4-dihydro-3(2H)-quinazolinyl) phénylcarbamate de tert-butyle

Le composé est préparé dans les mêmes conditions opératoires que pour l'Exemple 1 en partant du composé de la Préparation 2 avec le composé de la Préparation 14 "détritylé" au préalable par de l'acide chlorhydrique.
Spectrométrie de masse : [M+H]⁺= 462

### EXEMPLE 29 : 4-(1-[3-(1-Méthyl-1H-imidazol-5-yl)propyl]-2,4-dioxo-1,4-dihydro-3(2H)-quinazolinyl)phénylcarbamate de tert-butyle

Le composé est préparé dans les mêmes conditions opératoires que pour l'Exemple 22' en partant au Stade a des composés des Préparations 2 et 14 et en ajoutant au Stade b à la place du bromure de benzyle l'iodure de méthyle.
Spectrométrie de masse : [M+H]⁺= 476

### EXEMPLE 30 : 4-(1-[3-(1-Benzyl-1H-imidazol-5-yl)propyl]-2,4-dioxo-1,4-dihydro-3(2H)-quinazolinyl)phénylcarbamate de tert-butyle

Le composé est préparé dans les mêmes conditions opératoires que pour l'Exemple 22' en partant au Stade a des composés des Préparations 2 et 14.
Spectrométrie de masse : [M+H]⁺= 552

### EXEMPLE 31 : 4-(1-{3-[1-(4-Chlorobenzyl)-1H-imidazol-5-yl]propyl}-2,4-dioxo-1,4-dihydro-3(2H)-quinazolinyl)phénylcarbamate de tert-butyle

Le composé est préparé dans les mêmes conditions opératoires que pour l'Exemple 30 en utilisant comme réactif de départ, à la place du bromobenzyle, le 1-bromométhyl-4-chlorobenzène.
Spectrométrie de masse : [M+H]⁺= 586

### EXEMPLE 32 : 4-(1-{3-[1-(4-Cyanobenzyl)-1H-imidazol-5-yl]propyl}-2,4-dioxo-1,4-dihydro-3(2H)-quinazolinyl)phénylcarbamate de tert-butyle

Le composé est préparé dans les mêmes conditions opératoires que pour l'Exemple 30 en utilisant comme réactif de départ, à la place du bromobenzyle, le 1-bromométhyl-4-cyanobenzène.
Spectrométrie de masse : [M+H]⁺= 577

### EXEMPLE 33 : 4-(1-{3-[1-(4-Méthoxybenzyl)-1H-imidazol-5-yl]propyl}-2,4-dioxo-1,4-dihydro-3(2H)-quinazolinyl)phénylcarbamate de tert-butyle

Le composé est préparé dans les mêmes conditions opératoires que pour l'Exemple 30 en utilisant comme réactif de départ, à la place du bromobenzyle, le 1-chlorométhyl-4-méthoxybenzène.
Spectrométrie de masse : [M+H]⁺= 582

### EXEMPLE 34 : 4-(1-{3-[1-(4-Méthylbenzyl)-1H-imidazol-5-yl]propyl}-2,4-dioxo-1,4-dihydro-3(2H)-quinazolinyl)phénylcarbamate de tert-butyle

Le composé est préparé dans les mêmes conditions opératoires que pour l'Exemple 30 en utilisant comme réactif de départ, à la place du bromobenzyle, le 1-bromométhyl-4-toluène.
Spectrométrie de masse : [M+H]⁺= 566

### EXEMPLE 35 : 3-(4-Aminophényl)-1-{3-[1-(4-méthylbenzyl)-1H-imidazol-5-yl] propyl}-2,4(1H,3H)-quinazolinedione

Le composé est préparé à partir de la déprotection classique du groupement Boc du produit de l'Exemple 34 (acide chlorhydrique dans l'acétate d'éthyle).

### EXEMPLE 36 : Dichlorhydrate de 3-(4-aminophényl)-1-[3-(1H-imidazol-1-yl) propyl]-2,4(1H,3H)-quinazolinedione

Le composé est préparé à partir de la déprotection classique du groupement Boc du produit de l'Exemple 17 (acide chlorhydrique dans l'acétate d'éthyle).
Point de fusion : (Cap) Déc. >230 °C

| Microanalyses élémentaires : | | | | |
|---|---|---|---|---|
| | C | H | N | Cl |
| % Calculé | 55,30 | 4,88 | 16,13 | 16,32 |
| % Trouvé | 55,68 | 4,53 | 15,90 | 16,98 |

### EXEMPLE 37 : 3-(4-Chlorophényl)-1-[3-(1-méthyl-1H-imidazol-5-yl)propyl]-2,4(1H,3H)-quinazolinedione

Le composé est préparé dans les mêmes conditions opératoires que pour l'Exemple 29 en remplaçant le composé de la Préparation 2 par celui de la Préparation 15.
Spectrométrie de masse : [M+H]⁺= 395

### EXEMPLE 38 : 1-[3-(1-Benzyl-1H-imidazol-5-yl)propyl]-3-(4-chlorophényl)-2,4(1H,3H)-quinazolinedione

Le composé est préparé dans les mêmes conditions opératoires que pour l'Exemple 30 en remplaçant le composé de la Préparation 2 par celui de la Préparation 15.
Spectrométrie de masse : [M+H]⁺=471

### EXEMPLE 39 : 3-(4-Chlorophényl)-1-{3-[1-(4-méthylbenzyl)-1H-imidazol-5-yl] propyl}-2,4(1H,3H)-quinazolinedione

Le composé est préparé dans les mêmes conditions opératoires que pour l'Exemple 34 en remplaçant le composé de la Préparation 2 par celui de la Préparation 15.
Spectrométrie de masse : [M+H]⁺= 485

### EXEMPLE 40 : 3-(4-Chlorophényl)-1-{3-[1-(4-méthoxybenzyl)-1H-imidazol-5-yl] propyl}-2,4(1H,3H)-quinazolinedione

Le composé est préparé dans les mêmes conditions opératoires que pour l'Exemple 33 en remplaçant le composé de la Préparation 2 par celui de la Préparation 15.
Spectrométrie de masse : [M+H]⁺= 501,1

### EXEMPLE 41 : 3-(4-Chlorophényl)-1-{3-[1-(4-chlorobenzyl)-1H-imidazol-5-yl] propyl}-2,4(1H,3H)-quinazolinedione

Le composé est préparé dans les mêmes conditions opératoires que pour l'Exemple 31 en remplaçant le composé de la Préparation 2 par celui de la Préparation 15.
Spectrométrie de masse : [M+H]⁺= 505

### EXEMPLE 42 : 3-(4-Chlorophényl)-1-{3-[1-(4-cyanobenzyl)-1H-imidazol-5-yl] propyl}-2,4(1H,3H)-quinazolinedione

Le composé est préparé dans les mêmes conditions opératoires que pour l'Exemple 32 en remplaçant le composé de la Préparation 2 par celui de la Préparation 15.
Spectrométrie de masse : [M+H]⁺= 496

### EXEMPLE 43 : 3-(3-Bromophényl)-1-[3-(1H-imidazol-5-yl)propyl]-2,4(1H,3H)-quinazolinedione

Le composé est préparé dans les mêmes conditions opératoires que pour l'Exemple 21 en partant au Stade a du composé de la Préparation 1.
Spectrométrie de masse : [M+H]⁺= 425

### EXEMPLE 44 : 3-(3-Bromophényl)-1-[3-(1-méthyl-1H-imidazol-5-yl)propyl]-2,4(1H,3H)-quinazolinedione

Le composé est préparé dans les mêmes conditions opératoires que pour l'Exemple 22' en partant au Stade a des composés des Préparations 1 et 14 et en ajoutant au Stade b à la place du bromure de benzyle l'iodure de méthyle.
Spectrométrie de masse : [M+H]⁺= 439

### EXEMPLE 45 : 3-(3-Bromophényl)-1-{3-[1-(4-méthylbenzyl)-1H-imidazol-5-yl] propyl}-2,4(1H,3H)-quinazolinedione

Le composé est préparé dans les mêmes conditions opératoires que pour l'Exemple 44 en utilisant comme réactif de départ, à la place de l'iodure de méthyle, le 1-bromométhyl-4-toluène.
Spectrométrie de masse : [M+H]⁺= 529

### EXEMPLE 46 : 3-(3-Bromophényl)-1-{3-[1-(4-chlorobenzyl)-1H-imidazol-5-yl] propyl}-2,4(1H,3H)-quinazolinedione

Le composé est préparé dans les mêmes conditions opératoires que pour l'Exemple 44 en utilisant à la place de l'iodure de méthyle, le 1-bromométhyl-4-chlorobenzène.
Spectrométrie de masse : [M+H]⁺= 548,9

### EXEMPLE 47 : 3-(3-Bromophényl)-1-{3-[1-(4-cyanobenzyl)-1H-imidazol-5-yl] propyl}-2,4(1H,3H)-quinazolinedione

Le composé est préparé dans les mêmes conditions opératoires que pour l'Exemple 44 en utilisant à la place de l'iodure de méthyle, le 1-bromométhyl-4-cyanobenzène.
Spectrométrie de masse : [M+H]⁺= 540

### EXEMPLE 48 : 1-[3-(1-Benzyl-1H-imidazol-5-yl)propyl]-3-(3-bromophényl)-2,4(1H,3H)-quinazolinedione

Le composé est préparé dans les mêmes conditions opératoires que pour l'Exemple 44 en utilisant comme réactif de départ à la place de l'iodure de méthyle, le bromobenzyle.
Spectrométrie de masse : [M+H]⁺= 515

### EXEMPLE 49 : 3-(3-Bromophényl)-1-{3-[1-(4-méthoxybenzyl)-1H-imidazol-5-yl] propyl}-2,4(1H,3H)-quinazolinedione

Le composé est préparé dans les mêmes conditions opératoires que pour l'Exemple 44 en utilisant comme réactif de départ à la place de l'iodure de méthyle, le 1-chlorométhyl-4-méthoxybenzène.
Spectrométrie de masse : [M+H]⁺= 545,1

### EXEMPLE 50 : 3-(2-Méthoxyphényl)-1-[3-(1H-imidazol-5-yl)propyl]-2,4(1H,3H)-quinazolinedione

Le composé est préparé dans les mêmes conditions opératoires que pour l'Exemple 22' en partant au Stade a, des composés des Préparations 14 et 16 et en procédant au Stade b comme dans l'Exemple 21, Stade b.
Spectrométrie de masse : [M+H]⁺= 377,1

### EXEMPLE 51 : 3-(2-Méthoxyphényl)-1-[3-(1-méthyl-1H-imidazol-5-yl)propyl]-2,4(1H,3H)-quinazolinedione

Le composé est préparé dans les mêmes conditions opératoires que pour l'Exemple 22' en partant au Stade a, des composés des Préparations 14 et 16 et en utilisant au Stade b, à la place du bromure de benzyle l'iodure de méthyle.
Spectrométrie de masse : [M+H]⁺= 391,1

### EXEMPLE 52 : 1-[3-(1-Benzyl-1H-imidazol-5-yl)propyl]-3-(2-méthoxyphényl)-2,4(1H,3H)-quinazolinedione

Le composé est préparé dans les mêmes conditions opératoires que pour l'Exemple 51 en utilisant à la place de l'iodure de méthyle, le bromure de benzyle.
Spectrométrie de masse : [M+H]⁺= 466,7

### EXEMPLE 53 : 3-(2-Méthoxyphényl)-1-{3-[1-(4-méthylbenzyl)-1H-imidazol-5-yl] propyl}-2,4(1H,3H)-quinazolinedione

Le composé est préparé dans les mêmes conditions opératoires que pour l'Exemple 51 en utilisant à la place de l'iodure de méthyle, le 1-bromométhyl-4-toluène.
Spectrométrie de masse : [M+H]⁺= 481,1

### EXEMPLE 54 : 3-(2-Méthoxyphényl)-1-{3-[1-(4-méthoxybenzyl)-1H-imidazol-5-yl]propyl}-2,4(1H,3H)-quinazolinedione

Le composé est préparé dans les mêmes conditions opératoires que pour l'Exemple 51 en utilisant à la place de l'iodure de méthyle, le 1-chlorométhyl-4-méthoxybenzène.
Spectrométrie de masse : [M+H]⁺= 497,1

### EXEMPLE 55 : 3-(2-Méthoxyphényl)-1-{3-[1-(4-chlorobenzyl)-1H-imidazol-5-yl] propyl}-2,4(1H,3H)-quinazolinedione

Le composé est préparé dans les mêmes conditions opératoires que pour l'Exemple 51 en utilisant à la place de l'iodure de méthyle, le 1-bromométhyl-4-chlorobenzène.
Spectrométrie de masse : [M+H]⁺= 501,1

### EXEMPLE 56 : 3-(2-Méthoxyphényl)-1-{3-[1-(4-cyanobenzyl)-1H-imidazol-5-yl] propyl}-2,4(1H,3H)-quinazolinedione

Le composé est préparé dans les mêmes conditions opératoires que pour l'Exemple 51 en utilisant à la place de l'iodure de méthyle, le 1-bromométhyl-4-cyanobenzène.
Spectrométrie de masse : [M+H]⁺= 492,1

### EXEMPLE 57 : 3-(4-Chlorophényl)-1-[3-(1H-imidazol-5-yl)propyl]-2,4(1H,3H)-quinazolinedione

Le composé est préparé dans les mêmes conditions opératoires que pour l'Exemple 22' en partant au Stade a, des composés des Préparations 14 et 15 et en procédant au Stade b comme dans l'Exemple 21, Stade b.
Spectrométrie de masse : [M+H]⁺= 381

### EXEMPLE 58 : 1-{3-[1-(4-Méthylbenzyl)-1H-imidazol-5-yl]propyl}-3-phényl-2,4(1H,3H)-quinazolinedione

Le composé est préparé dans les mêmes conditions opératoires que pour l'Exemple 22' en utilisant au Stade b, à la place du bromure de benzyle, du 1-bromométhyl-4-toluène.
Spectrométrie de masse : [M+H]⁺= 451,1

### EXEMPLE 59 : 1-{3-[1-(4-Méthoxybenzyl)-1H-imidazol-5-yl]propyl}-3-phényl-2,4(1H,3H)-quinazolinedione

Le composé est préparé dans les mêmes conditions opératoires que pour l'Exemple 58 en en utilisant à la place du 1-bromométhyl-4-toluène, du 1-chlorométhyl-4-méthoxybenzène.
Spectrométrie de masse : [M+H]⁺= 467,1

### EXEMPLE 60 : 1-{3-[1-(4-Chlorobenzyl)-1H-imidazol-5-yl]propyl}-3-phényl-2,4(1H,3H)-quinazolinedione

Le composé est préparé dans les mêmes conditions opératoires que pour l'Exemple 58 en utilisant à la place du 1-bromométhyl-4-toluène, du 1-bromométhyl-4-chlorobenzène.
Spectrométrie de masse : [M+H]⁺= 471

### EXEMPLE 61 : 1-{3-[1-(4-Cyanobenzyl)-1H-imidazol-5-yl]propyl}-3-phényl-2,4(1H,3H)-quinazolinedione

Le composé est préparé dans les mêmes conditions opératoires que pour l'Exemple 58 en utilisant à la place du 1-bromométhyl-4-toluène, le 1-bromométhyl-4-cyanobenzène.
Spectrométrie de masse : [M+H]⁺= 462,1

### EXEMPLE 62 : 3-(2-Fluorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 17.
Spectrométrie de masse : [M+H]⁺= 365,1

### EXEMPLE 63 : 3-(2-Chlorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 18.
Spectrométrie de masse : [M+H]⁺= 381,1

### EXEMPLE 64 : 3-(2-Bromophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 19.
Spectrométrie de masse : [M+H]⁺= 425

### EXEMPLE 65 : 3-(2-Trifluorométhylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 20.
Spectrométrie de masse : [M+H]⁺= 415

### EXEMPLE 66 : 3-(2-Nitrophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 21.
Spectrométrie de masse : [M+H]⁺= 392

### EXEMPLE 67 : 3-(2-Anilinophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 22.
Spectrométrie de masse : [M+H]⁺= 438

### EXEMPLE 68 : 3-(2-Benzylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 23.
Spectrométrie de masse : [M+H]⁺= 437,1

### EXEMPLE 69 : 3-(2-Cyanophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 60.
Spectrométrie de masse : [M+H]⁺= 372,1

### EXEMPLE 70 : 3-(3-Fluorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 24.
Spectrométrie de masse : [M+H]⁺= 365,1

### EXEMPLE 71 : 3-(3-Chlorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 25.
Spectrométrie de masse : [M+H]⁺= 381

### EXEMPLE 72 : 3-(3-Trifluorométhylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 26.
Spectrométrie de masse : [M+H]⁺= 415

### EXEMPLE 73 : 3-(3-Nitrophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 27.
Spectrométrie de masse : [M+H]⁺= 392,1

### EXEMPLE 74 : 3-(3-Cyanophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 28.
Spectrométrie de masse : [M+H]⁺= 372,1

### EXEMPLE 75 : 3-(3-Méthoxyphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 29.
Spectrométrie de masse : [M+H]⁺= 377,1

### EXEMPLE 76 : 3-(3-Diméthylaminophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 30.
Spectrométrie de masse : [M+H]⁺= 390,1

### EXEMPLE 77 : 3-(3,5-Dichlorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 31.
Spectrométrie de masse : [M+H]⁺= 415

### EXEMPLE 78 : 3-[3,5-Bis(trifluorométhyl)phényl]-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 32.
Spectrométrie de masse : [M+H]⁺= 483

### EXEMPLE 79 : 3-(4-Fluorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 34.
Spectrométrie de masse : [M+H]⁺= 365,1

### EXEMPLE 80 : 3-(4-Chlorophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 15.
Spectrométrie de masse : [M+H]⁺= 381

### EXEMPLE 81 : 3-(4-Bromophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 35.
Spectrométrie de masse : [M+H]⁺= 425

### EXEMPLE 82 : 3-(4-Trifluorométhylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 36.
Spectrométrie de masse : [M+H]⁺= 415,1

### EXEMPLE 83 : 3-(4-Nitrophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 37.
Spectrométrie de masse : [M+H]⁺= 392,1

### EXEMPLE 84 : 3-(4-Cyanophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 38.
Spectrométrie de masse : [M+H]⁺= 372,1

### EXEMPLE 85 : 3-(4-Méthylphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 39.
Spectrométrie de masse : [M+H]⁺= 361

### EXEMPLE 86 : 3-(4-Méthoxyphényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 40.
Spectrométrie de masse : [M+H]⁺= 377

### EXEMPLE 87 : 3-(4-Diméthylaminophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 41.
Spectrométrie de masse : [M+H]⁺= 390,1

### EXEMPLE 88 : 3-(4-Anilinophényl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 42.
Spectrométrie de masse : [M+H]⁺= 438,1

### EXEMPLE 89 : 3-(2-Pyridyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 43.
Spectrométrie de masse : [M+H]⁺= 348,1

### EXEMPLE 90 : 3-(3-Pyridyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 44.
Spectrométrie de masse : [M+H]⁺= 348,1

### EXEMPLE 91 : 3-(4-Pyridyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 45.
Spectrométrie de masse : [M+H]⁺= 348,1

### EXEMPLE 92 : 3-Cyclohexyl-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 46.
Spectrométrie de masse : [M+H]⁺= 353

### EXEMPLE 93 : 3-Bicyclo[2.2.1]hept-2-yl-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 47.
Spectrométrie de masse : [M+H]⁺= 365

### EXEMPLE 94 : 3-(1,3-Benzodioxol-5-yl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 48.
Spectrométrie de masse : [M+H]⁺= 391,1

### EXEMPLE 95 : 3-(1-Naphtyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 49.
Spectrométrie de masse : [M+H]⁺= 397,1

### EXEMPLE 96 : 3-Benzyl-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 50.
Spectrométrie de masse : [M+H]⁺= 361,1

### EXEMPLE 97 : 3-(2-Phénéthyl)-1-[3-(1H-imidazol-1-yl)propyl] -2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 51.
Spectrométrie de masse : [M+H]⁺= 375,2

### EXEMPLE 98 : 3-(3-Phénylpropyl)-1-[3-(1H-imidazol-1-yl)propyl] -2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 52.
Spectrométrie de masse : [M+H]⁺= 389,2

### EXEMPLE 99 : 3-(4-Phénylbutyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 53.
Spectrométrie de masse : [M+H]⁺= 403,3

### EXEMPLE 100 : 3-(2-Pyridylméthyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 54.
Spectrométrie de masse : [M+H]⁺= 362,1

### EXEMPLE 101 : 3-(3-Pyridylméthyl)-1-[3-(1H-imidazol-1-yl)propyl] -2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 55.
Spectrométrie de masse : [M+H]⁺= 362,1

### EXEMPLE 102 : 3-(Cyclopropylméthyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 56.
Spectrométrie de masse : [M+H]⁺= 325,2

### EXEMPLE 103 : 3-(Adamantylméthyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 57.
Spectrométrie de masse : [M+H]⁺= 419

### EXEMPLE 104 : 3-(2-Oxo-2-phénéthyl)-1-[3-(1H-imidazol-1-yl)propyl]-2,4(1H,3H)-quinazolinedione

Le protocole expérimental est identique à celui de l'Exemple 1 en utilisant à la place de la 3-phényl-2,4(1*H*,3*H*)-quinazolinedione le composé de la Préparation 58.
Spectrométrie de masse : [M+H]⁺= 389,1

### EXEMPLE 105 : Bis trifluoroacétate de 1-[2-Benzyl-3-(1-méthyl-1H-imidazol-5-yl) propyl]-3-phényl-2,4(1H,3H)-quinazolinedione

Le protocole expérimental est identique à celui de l'Exemple 21 en utilisant au Stade a à la place du produit de la préparation 14 le 2-benzyl-3-(1-trityl-1*H*-imidazol-5-yl)-1-propanol, l'acidification au Stade b a été réalisée avec l'acide trifluoroacétique à la place de l'acide chlorhydrique suivie de la méthylation de l'imidazole suivant les mêmes conditions opératoires que celles de l'Exemple 23.

| Microanalyses élémentaires : | | | |
|---|---|---|---|
| | C | H | N |
| % Calculé | 56,64 | 4,16 | 8,26 |
| % Trouvé | 55,11 | 4,15 | 7,94 |

### EXEMPLE 106 : 1-[2-Phényl-3-(1-méthyl-1H-imidazol-5-yl)propyl]-3-phényl-2,4(1H,3H)-quinazolinedione

Le protocole expérimental est identique à celui de l'Exemple 21 en utilisant au Stade a à la place du produit de la préparation 14 le 2-phényl-3-(1-trityl-1*H*-imidazol-5-yl)-1-propanol suivi après le Stade b d'une méthylation de l'imidazole suivant les mêmes conditions opératoires que celles de l'Exemple 23.

| Microanalyses élémentaires : | | | |
|---|---|---|---|
| | C | H | N |
| % Calculé | 74,29 | 5,54 | 12,84 |
| % Trouvé | 73,94 | 5,34 | 12,70 |

### EXEMPLE 107 : 4-(1-[2-(4-Bromobenzyl)-3-(1H-imidazol-1-yl)propyl]-2,4-dioxo-1,4-dihydro-3(2H)-quinazolinyl)phénylcarbamate de tert-butyle

Le protocole expérimental est identique à celui de l'exemple 1 en utilisant à la place de la 3-phényl-2,4(1*H*,3*H*)-quinazolinedione le composé de la préparation 2 et à la place du produit de la préparation 6 celui de la préparation 61.
Spectrométrie de masse : [M+H]⁺ = 630

### EXEMPLE 108 : 4-[3-(3-Bromophényl)-2,4-dioxo-3,4-dihydro-1(2H)-quinazolinyl)-2-(1H-imidazol-1-ylméthyl)propyl]cyanophényle

Le protocole expérimental est identique à celui de l'exemple 1 en utilisant à la place de la 3-phényl-2,4(1*H*,3*H*)-quinazolinedione le composé de la préparation 1 et à la place du produit de la préparation 6 celui de la préparation 62.
Spectrométrie de masse : [M+H]⁺ = 540

### EXEMPLE 109 : 4-(1-[2-(4-Cyanobenzyl)-3-(1H-imidazol-1-yl)propyl]-2,4-dioxo-1,4-dihydro-3(2H)-quinazolinyl)phénylcarbamate de tert-butyle

Le protocole expérimental est identique à celui de l'exemple 1 en utilisant à la place de la 3-phényl-2,4(1*H*,3*H*)-quinazolinedione le composé de la préparation 2 et à la place du produit de la préparation 6 celui de la préparation 62.
Spectrométrie de masse : [M+H]⁺ = 577

### ETUDE PHARMACOLOGIQUE

### EXEMPLE A : Tests enzymatiques

Les deux enzymes FTase et GGTase-I ont été purifiées à partir de cerveaux de rat. Après broyage et centrifugation, le surnageant est précipité au sulfate d'ammonium à 30% et le surnageant correspondant soumis à une autre précipitation au sulfate d'ammonium à 50%. Le culot est alors passé sur une colonne de phényl agarose et les fractions collectées après élution au chlorure de sodium sont évaluées pour leur teneur enzymatique selon la méthode de "scintillation proximity assay" décrite ci-dessous. Les fractions correspondant à l'une ou l'autre des deux enzymes sont alors regroupées et congelées à -80°C jusqu'à utilisation.

Le dosage de l'activité enzymatique de la FTase est réalisé en plaques de 96 puits par la méthode radioactive de scintillation proximity assay. Le substrat accepteur composé de la séquence carboxyterminale de la lamine B (YRASNRSCAIM) couplée à la biotine est incubé en présence du substrat donneur radiomarqué (le [³H]farnésyl pyrophosphate), et de diverses concentrations de composés à tester dans le diméthylsulfoxyde (DMSO). La réaction est initiée à 37°C en ajoutant l'enzyme FTase pour une durée d'une heure, puis stoppée avec un tampon approprié contenant une suspension de billes imprégnées de scintillant. Ces billes sont de plus couplées à la streptavidine pour piéger, par couplage à la biotine, le peptide susceptible d'être farnésylé et mettre ainsi en contact le farnésyl radiomarqué avec le scintillant. Les plaques sont lues dans un compteur pour radioactivité et les données converties en pourcentages du contrôle pour exprimer les résultats sous forme de concentration du produit testé entraînant 50% d'inhibition de la farnésylation (IC50).
Pour la GGTase-I, un test équivalent a été utilisé en remplaçant le substrat accepteur par la séquence biotinylée TKCVIL et le substrat donneur par du [³H]géranylgéranyl pyrophosphate.

### Résultats :

Les composés de la présente invention possèdent des IC50 inférieures au micromolaire vis à vis de la FTase, révélant leur caractère de puissant inhibiteur de cet enzyme, et présentent une sélectivité importante par rapport à la GGTase-I, les IC50 étant alors supérieures au micromolaire.
A titre d'exemple, le composé de l'exemple 2 présente un IC50 de 19 nM vis-à-vis de la FTase.

### EXEMPLE B : Tests de prolifération cellulaire :

a) La lignée RAT2 de fibroblastes de rat et un transfectant correspondant à l'insertion du gène v-H-ras ont été utilisés pour tester la puissance cellulaire des produits revendiqués. Les cellules RAT2 permettent de caractériser la toxicité intrinsèque du produit testé, alors que les cellules transfectées qui exhibent une morphologie altérée et une vitesse de croissance plus rapide, servent à mesurer l'effet spécifique recherché sur la FTase intracellulaire.
   Les cellules parentales et transfectées sont ensemencées en plaques 96 puits pour la culture cellulaire en présence de milieu contenant 10% de sérum. Vingt-quatre heures après, les produits à tester sont ajoutés dans le même milieu sur une période de quatre jours et la quantité finale de cellules est estimée indirectement par la méthode de viabilité cellulaire au bromure de 3-(4,5-diméthylthiazol-2-yl)-2,5-diphényltétrazolium (MTT).
   *Résultats :*
   Un ralentissement de la croissance des cellules transfectées par v-H-ras est observé pour les composés de l'invention dans la gamme des dizaines de nanomolaire. Cet effet traduisant le retour des cellules transfectées aux caractéristiques de croissance de la lignée parentale, s'accompagne aussi d'une réversion de la morphologie des transfectants vers le phénotype parental (étalement et perte de réfringence). Plusieurs unités logarithmiques séparent cet effet spécifique de l'effet cytotoxique observé sur les cellules RAT2 dans la gamme des micromolaires, le différentiel le plus favorable étant d'au moins quatre unités pour les produits les plus actifs.
   A titre d'exemple, le composé de l'exemple 2 présente un IC50 d'inhibition de croissance de cellules transfectées par l'encogène v-H-ras de 9 nM.
b) Des tests complémentaires sur des lignées de carcinomes humains issues de biopsies cliniques sont effectués. Les lignées utilisées proviennent toutes de l'ATCC (American Type Culture Collection) et le test est réalisé en plaques 96 puits sur une durée de contact avec le produit correspondant à quatre temps de doublement.
   *Résultats :*
   Un dénombrement indirect par la méthode du MTT a permis de mettre en évidence une activité anti-proliférative avec des IC50s de l'ordre du micromolaire pour les composés de l'invention.

### EXEMPLE C : Composition pharmaceutique

| Formule de préparation pour 1000 comprimés dosés à 10 mg | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I): 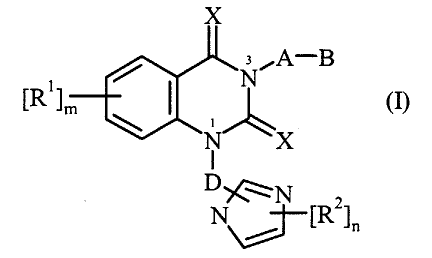 dans laquelle :
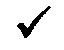 A représente une liaison, un groupement alkylène, alkénylène, alkynylène, T, *-A₁-T-, *-T-A₁-, *-A₁-T-A'₁- ou *-A₁-T-A'₁-T'- (dans lesquels T et T', identiques ou différents, représentent un groupement carbonyle, carbonyloxy, thio, sulfiriyle sulfonyle, oxy, amino, aminoalkyle, aminoaryle, carbonylamino, carbonylaminoalkyle, carbonylaminoaryle, oxycarbonyle, aminocarbonyle, aminoalkylcarbonyle, aminoarylcarbonyle, sulfonylamino, sulfonylaminoalkyle, sulfonylaminoaryle, aminosulfonyle, aminoalkylsulfonyle ou aminoarylsulfonyle ; A₁ et A'₁, identiques ou différents, représentent un groupement alkylène, alkénylène ou alkynylène ; et le symbole "*" représente le point de rattachement à l'atome d'azote N³ du cycle quinazoline),
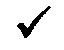 B représente un groupement alkyle éventuellement substitué, aryle éventuellement substitué, hétéroaryle éventuellement substitué, cycloalkyle éventuellement substitué, hétérocycloalkyle éventuellement substitué, arylaminoaryle éventuellement substitué ou arylalkylaryle éventuellement substitué,
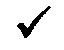 D représente un groupement alkylène dont un atome de carbone de la chaîne hydrocarbonée peut être substitué par un groupement alkyle éventuellement substitué, aryle éventuellement substitué, hétéroaryle éventuellement substitué, cycloalkyle éventuellement substitué, hétérocycloalkyle éventuellement substitué, arylalkyle éventuellement substitué, hétéroarylalkyle éventuellement substitué ou cycloalkylalkyle éventuellement substitué,
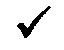 X représente un atome d'oxygène ou de soufre,
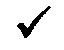 R¹ représente un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
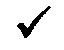 R² représente un atome d'hydrogène, un groupement alkyle, aryle, hétéroaryle, cycloalkyle, hétérocycloalkyle, arylalkyle, hétéroarylalkyle, cycloalkylalkyle ou hétérocycloalkylakyle, tous ces groupements pouvant éventuellement être substitués par un substituant choisi parmi un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle, alkoxycarbonylamino, arylamino éventuellement substitué ou arylalkyle éventuellement substitué,
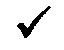 m représente un entier compris inclusivement entre 0 et 4,
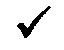 n représente un entier compris inclusivement entre 0 et 3,
leurs énantiomères, diastéréoisomères, ainsi que leurs sels d'addition à un acide ou à une base pharmaceutiquement acceptable,
étant entendu que :
- pour m supérieur à 1, les groupements R¹ peuvent être identiques ou différents les uns des autres,
- pour n supérieur à 1, les groupements R² peuvent être identiques ou différents les uns des autres,
- lorsque les deux atomes d'azote du groupement imidazolyle sont substitués, le groupement imidazolyle devient un groupement cationique imidazolinium,
- le terme alkyle désigne une chaîne hydrocarbonée, linéaire ou ramifiée, contenant de 1 à 6 atomes de carbone,
- le terme alkoxy désigne un groupement alkyle-oxy, linéaire ou ramifié, contenant de 1 à 6 atomes de carbone,
- le terme alkylène désigne une chaîne hydrocarbonée bivalente, linéaire ou ramifiée, contenant de 1 à 6 atomes de carbone,
- le terme alkénylène désigne une chaîne hydrocarbonée bivalente linéaire ou ramifiée, contenant de 1 à 3 doubles liaisons et de 2 à 6 atomes de carbone,
- le terme alkynylène désigne une chaîne hydrocarbonée bivalente linéaire ou ramifiée, contenant de 1 à 3 triples liaisons et de 2 à 6 atomes de carbone,
- le terme cycloalkyle désigne un groupement mono ou polycyclique, saturé ou partiellement saturé, contenant de 3 à 10 atomes de carbone,
- le terme hétérocycloalkyle désigne un groupement mono ou polycyclique saturé ou partiellement insaturé de 5 à 7 chaînons contenant de 1 à 3 hétéroatomes choisis parmi l'azote, l'oxygène et le soufre,
- le terme aryle désigne un groupement phényle, naphtyle ou biphényle,
- le terme hétéroaryle désigne un groupement mono ou bicyclique, aromatique ou contenant au moins un cycle aromatique, de 5 à 11 chaînons, contenant de 1 à 3 hétéroatomes choisis parmi l'azote, l'oxygène ou le soufre,
- le terme éventuellement substitué, affecté à l'expression alkyle, signifie qu'un à trois atomes de carbone de la chaîne hydrocarbonée peuvent être substitués par un à trois substituants, identiques ou différents, choisis parmi atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
- le terme éventuellement substitué, sauf indication contraire, affecté aux expressions aryle, hétéroaryle, cycloalkyle, hétérocycloalkyle, arylalkylaryle et arylaminoaryle, signifie que la ou les parties cycliques de ces groupements peuvent être substituées par un à trois substituants, identiques ou différents, choisi parmi un atome d'halogène, un groupement alkyle, alkoxy, hydroxy, mercapto, cyano, amino, alkylamino, dialkylamino, nitro, perhalogénoalkyle, carboxy, alkoxycarbonyle, aminocarbonyle, alkylaminocarbonyle, dialkylaminocarbonyle, carbamoyle ou alkoxycarbonylamino,
- le terme perhalogénoalkyle désigne un groupement méthyle, éthyle, propyle ou butyle substitué par de 1 à 9 atomes d'halogènes.

2. Composés de formule (I), selon la revendication 1, pour lesquels A représente une liaison, un groupement sulfonyle ou alkylène, leurs énantiomères, diastéréoisomères, ainsi que leurs sels d'addition à un acide ou à une base pharmaceutiquement acceptable.

3. Composés de formule (I), selon l'une quelconque des revendications 1 et 2, pour lesquels B représente un groupement aryle éventuellement substitué ou hétéroaryle éventuellement substitué, leurs énantiomères, diastéréoisomères, ainsi que leurs sels d'addition à un acide ou à une base pharmaceutiquement acceptable.

4. Composé de formule (I), selon l'une quelconque des revendications 1 à 3, pour lesquels m vaut 0, leurs énantiomères, diastéréoisomères, ainsi que leurs sels d'addition à un acide ou à une base pharmaceutiquement acceptable.

5. Composés de formule (I), selon l'une quelconque des revendications 1 à 4, pour lesquels X représente un atome d'oxygène, leurs énantiomères, diastéréoisomères, ainsi que leurs sels d'addition à un acide ou à une base pharmaceutiquement acceptable.

6. Composés de formule (I), selon l'une quelconque des revendications 1 à 5, pour lesquels D représente un groupement alkylène substitué par un groupement aryle éventuellement substitué ou arylalkyle éventuellement substitué, leurs énantiomères, diastéréoisomères, ainsi que leurs sels d'addition à un acide ou à une base pharmaceutiquement acceptable.

7. Composés de formule (I), selon l'une quelconque des revendications 1 à 6, pour lesquels R² représente un atome d'hydrogène, un groupement alkyle ou arylalkyle éventuellement substitué, leurs énantiomères, diastéréoisomères, ainsi que leurs sels d'addition à un acide ou à une base pharmaceutiquement acceptable.

8. Composés de formule (I), selon l'une quelconque des revendications 1 à 7, pour lesquels n vaut 0, 1 ou 2, leurs énantiomères, diastéréoisomères, ainsi que leurs sels d'addition à un acide ou à une base pharmaceutiquement acceptable.

9. Composés de formule (I), selon l'une quelconque des revendications 1 à 8, pour lesquels m vaut 0, X représente un atome d'oxygène, A représente une liaison, un groupement sulfonyle ou alkylène, B représente un groupement phényle éventuellement substitué, benzylphényle, pyridyle, anilinophényle ou tiényle, D représente un groupement alkylène substitué ou non par un groupement phényle éventuellement substitué ou phénylméthyle éventuellement substitué, n vaut 0, 1 ou 2 et R² représente un groupement alkyle ou arylalkyle éventuellement substitué, leurs énantiomères, diastéréoisomères, ainsi que leurs sels d'addition à un acide ou à une base pharmaceutiquement acceptable.

10. Composés de formule (I), selon l'une quelconque des revendications 1 à 8, pour lesquels m vaut 0, X représente un atome d'oxygène, A représente une liaison, un groupement sulfonyle ou alkylène, B représente un groupement phényle éventuellement substitué, benzylphényle, pyridyle, anilinophényle ou tiényle, D représente un groupement alkylène, substitué ou non par un groupement phényle éventuellement substitué ou phénylméthyle éventuellement substitué, relié à un des atomes d'azote de l'imidazolyle, n vaut 0, 1 ou 2 et R² représente un groupement alkyle ou arylalkyle éventuellement substitué, leurs énantiomères, diastéréoisomères, ainsi que leurs sels d'addition à un acide ou à une base pharmaceutiquement acceptable.

11. Composé de formule (I), selon l'une quelconque des revendications 1 à 10 qui est le 4-(1-[2-benzyl-3-(1*H*-imidazol-1-yl)propyl]-2,4-dioxo-1,4-dihydro-3(2*H*)-quinazolinyl) phénylcarbamate de *tert*-butyle.

12. Composé de formule (I), selon l'une quelconque des revendications 1 à 10 qui est le 1-[2-benzyl-3-(1*H*-imidazol-1-yl)propyl]-3-(3-bromophényl)- 2,4(1*H*,3*H*)-quinazolinedione.

13. Composé de formule (I), selon l'une quelconque des revendications 1 à 9 qui est le 3-(3-bromophényl)-1-{3-[1-(4-cyanobenzyl)-1*H*-imidazol-5-yl]propyl}-2,4(1*H*,3*H*)-quinazolinedione.

14. Composé de formule (I), selon l'une quelconque des revendications 1 à 9 qui est le bis trifluoroacétate de 1-[2-benzyl-3-(1-méthyl-1*H*-imidazol-5-yl)propyl]-3-phényl-2,4( 1*H*,3*H*)-quinazolinedione.

15. Procédé de préparation des composés de formule (I), selon la revendication 1, **caractérisé en ce que** nous utilisons comme produit de départ un composé de formule (II) : 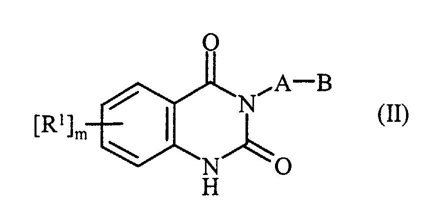 dans laquelle R¹, A, B et m sont tels que définis dans la formule (I) qui se condense avec le dérivé alcoolique de formule (III) : 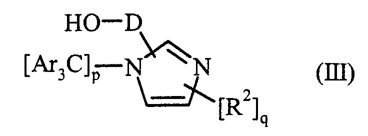 dans laquelle Ar représente un groupement phényle éventuellement substitué, q est un entier compris inclusivement entre 0 et 2, R² et D sont tels que définis dans la formule (I) et p est un entier compris inclusivement entre 0 et 1,
pour conduire, en présence de dialkylazodicarboxylate et de triarylphosphine, au composé de formule (IV) : 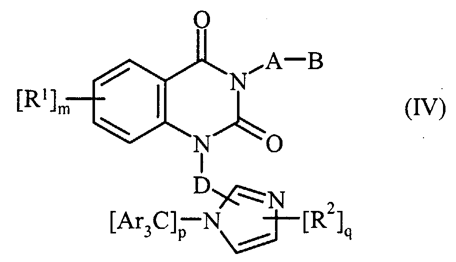 dans laquelle Ar, R¹, R², A, B, D, m, p et q sont tels que définis précédemment,
qui est soumis à l'action d'agent de thionation de fonctions cétones, pour conduire au composé de formule (V) : 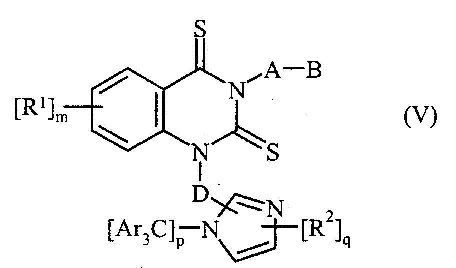 dans laquelle Ar, R¹, R², A, B, D, m, p et q sont tels que définis précédemment,
les composés de formules (IV) et (V) sont représentés par la formule (VI) : 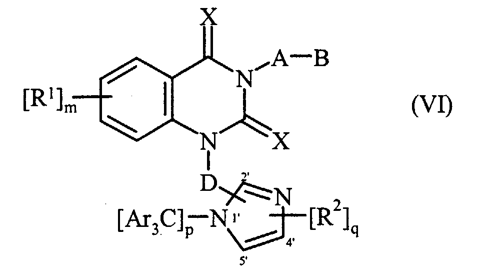 dans laquelle Ar, R¹, R², A, B, D, m, p, q sont tels que définis précédemment et X est tel que défini dans la formule (I),
* composé de formule (VI), lorsque D est relié au groupement imidazolyle par l'atome d'azote N_{1'} (où p vaut 0) correspond au composé de formule (I/a), cas particulier des composés de formule (I) : 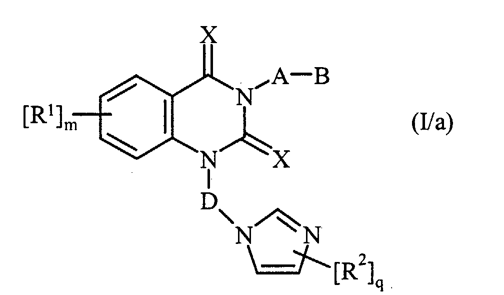
dans laquelle R¹, R², A, B, D, X, m et q sont tels que définis précédemment,
qui peut être soumis au réactif suivant : Hal-R³ dans lequel Hal représente un atome d'halogène et R³ peut prendre toutes les valeurs de R² à l'exception du groupement aryle et hétéroaryle,
pour conduire au composé cationique (I/b), cas particulier des composés de formule (I) : 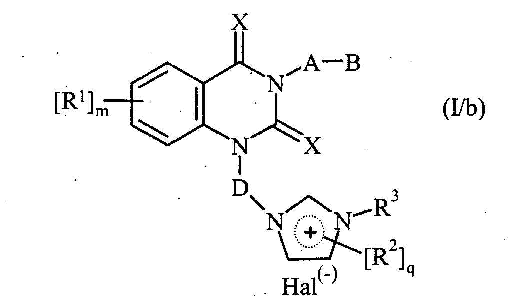 dans laquelle R¹, R², R³, A, B, D, X, m, q sont tels que définis précédemment et Hal⁽⁻⁾ représente l'anion correspondant à l'atome d'halogène Hal,
* composé de formule (VI), lorsque D est relié au groupement imidazolyle par les atomes de carbone C_{2'}, C_{4'} et C_{5'} (où p vaut 1) correspond au composé de formule (VII) : 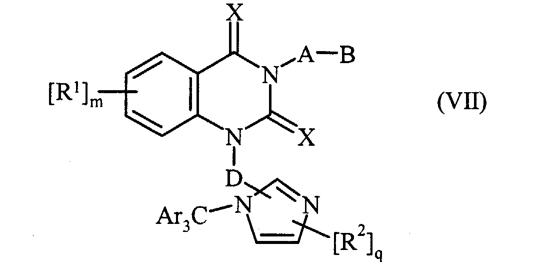
dans laquelle Ar, R¹, R², A, B, D, X, m et q sont tels que définis précédemment, qui peut :
→ **soit** être soumis à l'action d'un acide tel que l'acide chlorhydrique par exemple pour obtenir le composé déprotégé (I/c), cas particulier des composés de formule (I): 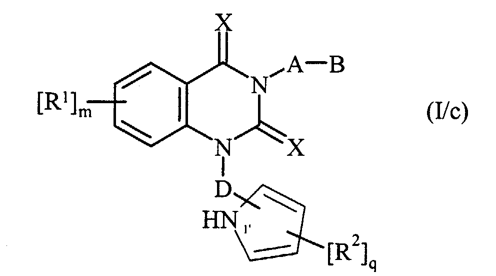
dans laquelle R¹, R², A, B, D, X, m et q sont tels que définis précédemment,
dont l'atome d'azote N_{1'} du groupement imidazolyle peut être substitué, en présence éventuellement d'un catalyseur approprié, par le réactif suivant : Hal-R⁴, dans lequel Hal est tel que défini précédemment et R⁴ peut prendre toutes les valeurs de R² à l'exception de l'atome d'hydrogène, des groupements aryle et hétéroaryle, pour conduire en milieu alcalin au composé de formule (I/d), cas particulier des composés de formule (I) : 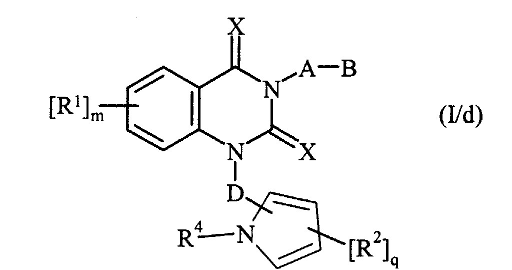 dans laquelle R¹, R², R⁴, A, B, D, X, m et q sont tels que définis précédemment,
→ **soit** réagir avec l'agent alkylant suivant : Hal-R⁵, dans lequel Hal représente un atome d'halogène et R⁵ peut prendre toutes les valeurs de R² à l'exception de l'atome d'hydrogène, des groupements aryle et hétéroaryle, pour conduire au composé cationique de formule (VIII) : 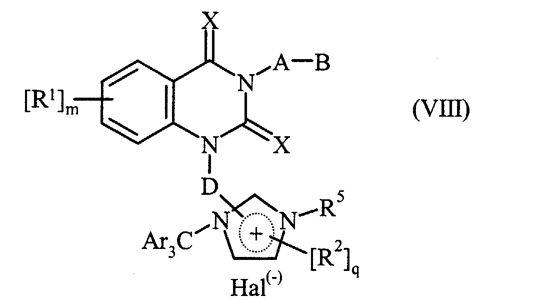
dans laquelle Ar, R¹, R², R⁵, A, B, D, X, m, Hal⁽⁻⁾ et q sont tels que définis précédemment,
qui est déprotégé, en chauffant dans le méthanol, pour conduire au composé de formule (I/e), cas particulier des composés de formule (I): 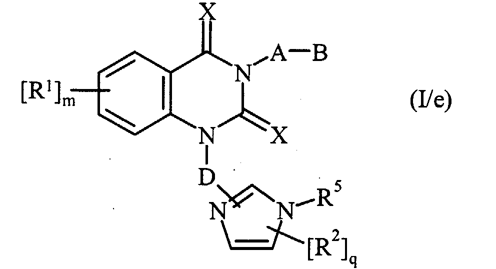 dans laquelle R¹, R², R³, A, B, D, X, m et q sont tels que définis précédemment,
l'ensemble des composés de formule (I/a) à (I/e) formant l'ensemble des composés de formule (I),
- qui peuvent être, le cas échéant, purifiés selon une technique classique de purification,
- dont les isomères (diastéréoisomères, énantiomères et isomères géométriques) sont éventuellement séparés par une technique classique de séparation,
- dont les intermédiaires peuvent être le cas échéant, protégés et déprotégés au cours de la synthèse pour faciliter l'accès aux produits recherchés,
- que l'on transforme, si on le souhaite, en leurs sels d'addition à un acide ou à une base pharmaceutiquement acceptable.

16. Compositions pharmaceutiques contenant comme principe actif au moins un composé selon l'une quelconque des revendications 1 à 14, en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications à 1 à 14 utiles pour la fabrication de médicaments utiles dans le traitement des maladies cancéreuses.

18. Compositions pharmaceutiques selon la revendication 16 contenant au moins un principe actif selon l'une quelconque des revendications 1 à 14 utiles pour la fabrication de médicaments utiles dans le traitement de la resténose après angioplastie ou chirurgie vasculaire, et de la neurofibromatose de type I.
